# EUROPÄISCHE PATENTSCHRIFT

(11) **EP 1 853 582 B1**
(45) Veröffentlichungstag und Bekanntmachung des Hinweises auf die Patenterteilung: **21.10.2009**
(21) Anmeldenummer: 06706932.8
(22) Anmeldetag: 14.02.2006
(51) Int. Cl.: C07D 401/12, C07D 233/90, A61K 31/4178, A61K 31/4164, A61P 31/12

(54) **HETEROCYCLYLAMID-SUBSTITUIERTE IMIDAZOLE**
HETEROCYCLYLAMIDE-SUBSTITUTED IMIDAZOLES
IMIDAZOLES SUBSTITUES PAR HETEROCYCLYLAMIDE

(30) Priorität: 23.02.2005 DE 102005008183
(43) Veröffentlichungstag der Anmeldung: 14.11.2007
(73) Patentinhaber: AiCuris GmbH & Co. KG, 42117 Wuppertal (DE)
(72) Erfinder: ZIMMERMANN, Holger, 42111 Wuppertal (DE); BRÜCKNER, David, 45128 Essen (DE); HENNINGER, Kerstin, 42115 Wuppertal (DE); ROSENTRETER, Ulrich, 31619 Binnen (DE); HENDRIX, Martin, 51519 Odenthal (DE); KELDENICH, Jörg, 42113 Wuppertal (DE); LANG, Dieter, 42553 Velbert (DE); RADTKE, Martin, 40699 Erkrath (DE); PAULSEN, Daniela, 42105 Wuppertal (DE); KERN, Armin, 42109 Wuppertal (DE)
(74) Vertreter: Weller, Wolfgang
(86) Internationale Anmeldenummer: PCT/EP2006/001325
(87) Internationale Veröffentlichungsnummer: WO 2006/089664

(56) Entgegenhaltungen:
- WO-A-98/52558
- WO-A-20/04052852
- WO-A-20/05092865
- CHAIMBAULT C ET AL: "SYNTHESIS, ANTITUMOUR AND ANTI-HIV SCREENING OF OXAZOLIDINES AND OXAZOLIDINONES DERIVATIVES" PHARMACY AND PHARMACOLOGY COMMUNICATIONS, LONDON, GB, Bd. 5, Nr. 3, März 1999 (1999-03), Seiten 211-215, XP009026199 ISSN: 1460-8081

## Beschreibung

Die Erfindung betrifft Heterocyclylamid-substituierte Imidazole und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Verwendung als antivirale Mittel, insbesondere gegen Cytomegalieviren.

WO 99/23091 beschreibt aromatische heterocyclische Verbindungen als antiinflammatorische Mittel, die unter anderem auch zur Behandlung von viralen Infektionen geeignet sein können.

Auf dem Markt sind zwar strukturell andersartige antiviral wirkende Mittel vorhanden, aber die gegenwärtig verfügbaren Therapien mit Ganciclovir, Valganciclovir, Foscarnet und Cidofovir sind mit schweren Nebenwirkungen verbunden, z. B. Nephrotoxizität, Neutropenie oder Thrombozytopenie. Zudem kann es regelmäßig zu einer Resistenzentwicklung kommen. Neue Mittel für eine wirksame Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen substituierten Imidazole antiviral hochwirksam sind.

Gegenstand der vorliegenden Erfindung sind Verbindungen der Formel 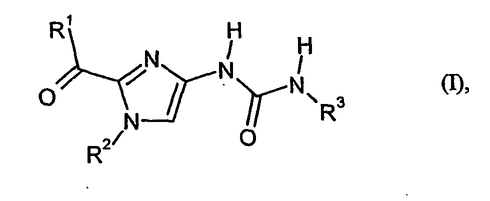 in welcher
- R¹: für eine Gruppe der Formel 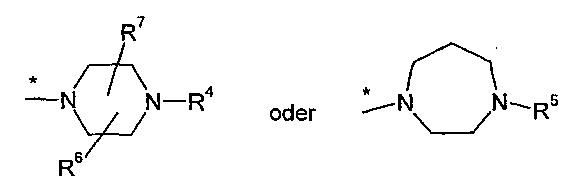 steht,
wobei
* für die Anknüpfstelle an die Carbonyl-Gruppe steht,
R⁴ für Phenyl oder 5- oder 6-gliedriges Heteroaryl steht,
worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
R⁵ für Phenyl oder 5- oder 6-gliedriges Heteroaryl steht,
worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl, und
R⁶ und R⁷ unabhängig voneinander für Wasserstoff, Methyl oder Ethyl stehen,

- R²: für C₁-C₆-Alkyl steht,
wobei Alkyl substituiert sein kann mit einem Substituenten, wobei der Substituent ausgewählt wird aus der Gruppe, bestehend aus C₃-C₆-Cycloalkyl, C₆-C₁₀-Aryl und 5- oder 6-gliedriges Heteroaryl,
worin Cycloalkyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluor-methoxy, Trifluormethylthio, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,

- R³: für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, Monofluormethoxy, Trifluormethylthio, C₁-C₆-Alkyl und C₁-C₆-Alkoxy,
und ihre Salze, ihre Solvate und die Solvate ihrer Salze.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkoxycarbonyl und Alkylaminocarbonyl stehen für einen linearen oder verzweigten Alkylrest mit in der Regel 1 bis 6 ("C₁-C₆-Alkyl"), vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N*,*N*-Dimethylamino, *N*,*N-*Diethylamino, *N*-Ethyl-*N*-methylamino, *N*-Methyl-*N*-n-propylamino, *N*-Isopropyl-*N-*n-propylamino, *N*-t-Butyl-*N*-methylamino, *N*-Ethyl-*N*-n-pentylamino und *N*-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, tert.-Butylaminocarbonyl, n-Pentylaminocarbonyl, n-Hexylaminocarbonyl, *N*,*N*-Dimethylaminocarbonyl, *N*,*N*-Diethylaminocarbonyl, *N*-Ethyl-*N*-methylaminocarbonyl, *N*-Methyl-*N*-n-propylaminocarbonyl, *N*-Isopropyl-*N*-n-propylaminocarbonyl, *N*-tert.-Butyl-*N*-methylaminocarbonyl, *N*-Ethyl-*N*-n-pentylamino-carbonyl und *N*-n-Hexyl-*N*-methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

Aryl steht für einen mono- oder bicyclischen aromatischen, carbocyclischen Rest mit in der Regel 6 bis 10 Kohlenstoffatomen; beispielhaft und vorzugsweise für Phenyl und Naphthyl.

5- oder 6-gliedriges Heteroaryl steht im Rahmen der Erfindung im allgemeinen für einen aromatischen, monocyclischen Rest mit 5 oder 6 Ringatomen und bis zu 4 Heteroatomen aus der Reihe S, O und/oder N. Der Heteroarylrest kann über ein Kohlenstoff oder Heteroatom gebunden sein. Beispielsweise und vorzugsweise seien genannt: Thienyl, Furyl, Pyrrolyl, Thiazolyl, Oxazolyl, Pyrazolyl, Imidazolyl, Pyridyl, Pyrimidyl und Pyridazinyl.

Cycloalkyl steht für eine Cycloalkylgruppe mit in der Regel 3 bis 8, bevorzugt 3 bis 6 Kohlenstoffatomen, beispielhaft und vorzugsweise für Cyclopropyl, Cyclobutyl, Cyclopentyl, Cyclohexyl und Cycloheptyl.

Halogen steht für Fluor, Chlor, Brom und Jod.

Bevorzugt im Rahmen der vorliegenden Erfindung sind Verbindungen der Formel (I),
in welcher
- R¹: für eine Gruppe der Formel 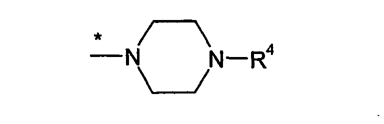 steht,
wobei
* für die Anknüpfstelle an die Carbonyl-Gruppe steht,
R⁴ für Phenyl oder 5- oder 6-gliedriges Heteroaryl steht,
worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, C₁-C₆-Alkyl, C₁-C₆-Alkoxy, Hydroxycarbonyl, C₁-C₆-Alkoxycarbonyl, Amino, C₁-C₆-AAylamino, Aminocarbonyl und C₁-C₆-Alkylaminocarbonyl,
- R²: für C₁-C₆-Alkyl steht,
wobei Alkyl substituiert sein kann mit einem Substituenten, wobei der Substituent ausgewählt wird aus der Gruppe, bestehend aus C₃-C₆-Cycloalkyl und Phenyl,
worin Cycloalkyl und Phenyl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
- R³: für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, Monofluormethoxy, Trifluormethylthio, C₁-C₆-Alkyl und C₁-C₆-Alkoxy,
und ihre Salze, ihre Solvate und die Solvate ihrer Salze.

Bevorzugt im Rahmen der vorliegenden Erfindung sind auch Verbindungen der Formel (I),
in welcher
- R¹: für eine Gruppe der Formel 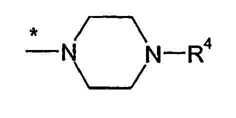 steht,
wobei
* für die Anknüpfstelle an die Carbonyl-Gruppe steht,
R⁴ für Phenyl oder Pyridyl steht,
worin Phenyl und Pyridyl substituiert sein können mit 1 bis 3 Substituenten, wobei die Substituenten unabhängig voneinander ausgewählt werden aus der Gruppe, bestehend aus Halogen, Nitro, Cyano, Trifluormethyl, Difluormethyl, Trifluormethoxy, Difluormethoxy, Monofluormethoxy, C₁-C₄-Alkyl und C₁-C₄-Alkoxy,
- R²: für Methyl, Ethyl oder n-Butyl steht,
wobei Methyl, Ethyl und n-Butyl substituiert sein können mit einem Substituenten, wobei der Substituent ausgewählt wird aus der Gruppe, bestehend aus Cyclopropyl und Phenyl,
worin Phenyl substituiert sein kann mit einem Substituenten Trifluormethyl,
- R³: für Phenyl steht,
wobei Phenyl substituiert sein kann mit 1 bis 3 Substituenten, wobei die Substituenten unabhängig voneinander ausgewählt werden aus der Gruppe, bestehend aus Fluor, Chlor, Trifluormethoxy, Difluormethoxy, Trifluormethylthio und Methyl,
und ihre Salze, ihre Solvate und die Solvate ihrer Salze.

Bevorzugt im Rahmen der vorliegenden Erfindung sind auch Verbindungen der Formel (I), in welcher R¹ für eine Gruppe der Formel 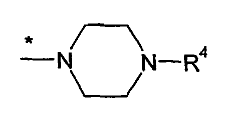 steht,
wobei
- *: für die Anknüpfstelle an die Carbonyl-Gruppe steht, und
- R⁴: für Phenyl oder Pyridyl steht,
worin Phenyl und Pyridyl substituiert sein können mit 1 bis 3 Substituenten, wobei die Substituenten unabhängig voneinander ausgewählt werden aus der Gruppe, bestehend aus Halogen, Nitro, Cyano, Trifluormethyl, Difluormethyl, Trifluormethoxy, Difluormethoxy, Monofluormethoxy, C₁-C₄-Alkyl und C₁-C₄-Alkoxy.
Bevorzugt im Rahmen der vorliegenden Erfindung sind auch Verbindungen der Formel (I), in welcher R³ für Phenyl steht, wobei Phenyl substituiert sein kann mit 1 bis 3 Substituenten, wobei die Substituenten unabhängig voneinander ausgewählt werden aus der Gruppe, bestehend aus Fluor, Chlor, Trifluormethoxy, Difluormethoxy, Trifluormethylthio und Methyl.

Gegenstand der Erfindung ist weiterhin ein Verfahren zur Herstellung der Verbindungen der Formel (I), wobei
nach Verfahren [A] Verbindungen der Formel 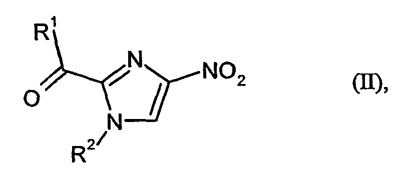 in welcher
R¹ und R² die oben angegebene Bedeutung haben,
in der ersten Stufe mit einem Reduktionsmittel und in der zweiten Stufe in Gegenwart eines Kohlensäurederivates mit Verbindungen der Formel

H₂N—R³ (III),

in welcher
R³ die oben angegebene Bedeutung hat,
oder
nach Verfahren [B] Verbindungen der Formel (II) in der ersten Stufe mit einem Reduktionsmittel und in der zweiten Stufe mit Verbindungen der Formel

OCN-R³ (IV),

in welcher
- R³: die oben angegebene Bedeutung hat,
oder
nach Verfahren [C] Verbindungen der Formel 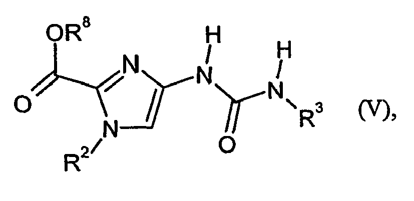 in welcher
- R² und R³: die oben angegebene Bedeutung haben, und
- R⁸: für Methyl oder Ethyl steht,
in der ersten Stufe mit einer Base und in der zweiten Stufe mit Verbindungen der Formel

R¹-H (VI),

in welcher
- R¹: die oben angegebene Bedeutung hat,
in Gegenwart von Dehydratisierungsreagenzien umgesetzt werden.

Die Verbindungen der Formeln (III), (IV) und (VI) sind bekannt oder lassen sich nach bekannten Verfahren aus den entsprechenden Edukten synthetisieren.

Für Verfahren [A] und [B] 1. Stufe gilt:

Die Umsetzung erfolgt im Allgemeinen in inerten Lösungsmitteln, bevorzugt in einem Temperaturbereich von 0°C bis zum Rückfluss der Lösungsmittel bei Normaldruck bis 3 bar.

Reduktionsmittel sind beispielsweise Palladium auf Aktivkohle und Wasserstoff, Ameisensäure/Triethylamin/Palladium auf Aktivkohle, Zink, Zink/Salzsäure, Eisen, Eisen/Salzsäure, Eisen(II)sulfat/Salzsäure, Natriumsulfid, Natriumdisulfid Natriumdithionit, Ammoniumpolysulfid, Natriumborhydrid/Nickelchlorid, Zinndichlorid, Titantrichlorid oder Raney-Nickel und wässrige Hydrazin-Lösung, bevorzugt ist Raney-Nickel und wässrige Hydrazin-Lösung, Palladium auf Aktivkohle und Wasserstoff oder Ameisensäure/Triethylamin/Palladium auf Aktivkohle.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, im Falle von wassermischbaren Lösungsmitteln auch Gemische derselben mit Wasser, als Lösungsmittel ist bevorzugt Methanol, Ethanol, iso-Propanol oder im Falle von Raney-Nickel und wässrige Hydrazin-Lösung Tetrahydrofuran.

Für Verfahren [A] 2. Stufe gilt:

Die Umsetzung erfolgt im Allgemeinen in inerten Lösungsmitteln, bevorzugt in einem Temperaturbereich von Raumtemperatur bis 40°C bei Normaldruck.

Kohlensäurederivate sind beispielsweise N,N-Carbonyldiimidazol, Phosgen, Diphosgen, Triphosgen, Chlorameisensäurephenylester oder Chlorameisensäure-4-nitrophenylester, bevorzugt ist N,N-Carbonyldiimidazol.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, im Falle von wassermischbaren Lösungsmitteln auch Gemische derselben mit Wasser, bevorzugt ist Dimethylsulfoxid.

Für Verfahren [B] 2. Stufe gilt:

Die Umsetzung erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, bevorzugt sind Tetrahydrofuran oder Methylenchlorid.

Basen sind beispielsweise Alkalicarbonate wie Cäsiumcarbonat, Natrium- oder Kaliumcarbonat, oder Kalium-tert.-butylat, oder andere Basen wie Natriumhydrid, DBU, Triethylamin oder Diisopropylethylamin, bevorzugt Triethylamin.

Für Verfahren [C] 1. Stufe gilt:

Die Umsetzung erfolgt im Allgemeinen in inerten Lösungsmitteln, bevorzugt in einem Temperaturbereich von 0°C bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt Natriumhydroxid.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Gemische von Lösungsmitteln mit Wasser, als Lösungsmittel ist bevorzugt ein Gemisch aus Ethanol und Wasser.

Für Verfahren [C] 2. Stufe gilt:

Die Umsetzung erfolgt im Allgemeinen in inerten Lösungsmitteln, gegebenenfalls in Gegenwart einer Base, bevorzugt in einem Temperaturbereich von -70°C bis 40°C bei Normaldruck.

Als Dehydratisierungsreagenzien eignen sich hierbei beispielsweise Carbodiimide wie z.B. N,N'-Diethyl-, N,N,'-Dipropyl-, N,N'-Diisopropyl-, N,N'-Dicyclohexylcarbodiimid, N-(3-Dimethyl-aminoisopropyl)-N-ethylcarbodümid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluorophosphat (BOP), oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin, oder DBU, DBN, Pyridin, bevorzugt ist Triethylamin.

Vorzugsweise wird die Kondensation mit Carbonyldiimidazol durchgeführt.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, im Falle von wassermischbaren Lösungsmitteln auch Gemische derselben mit Wasser, bevorzugt ist Dimethylformamid.

Die Verbindungen der Formel (II) sind bekannt oder können hergestellt werden, indem Verbindungen der Formel 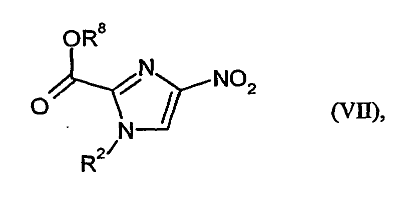 in welcher
- R²: die oben angegebene Bedeutung hat, und
- R⁸: für Methyl oder Ethyl steht,
in der ersten Stufe mit einer Base und in der zweiten Stufe mit Verbindungen der Formel (VI), in Gegenwart von Dehydratisierungsreagenzien umgesetzt werden.

Die Umsetzung erfogt wie in Verfahren [C] beschrieben.

Die Verbindungen der Formel (VII) sind bekannt oder können hergestellt werden, indem Verbindungen der Formel 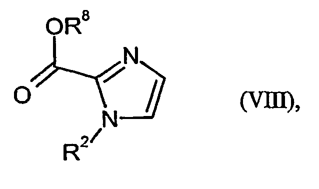 in welcher
- R²: die oben angegebene Bedeutung hat, und
- R⁸: für Methyl oder Ethyl steht,
mit rauchender Salpetersäure, konzentrierter Salpetersäure, Nitriersäure oder anderen Mischungsverhältnissen von Schwefelsäure und Salpetersäure, gegebenenfalls in Acetanhydrid als Lösungsmittel, bevorzugt in einem Temperaturbereich von Raumtemperatur bis 60°C bei Normaldruck, umgesetzt werden.

Die Verbindungen der Formel (V) sind bekannt oder können hergestellt werden, indem Verbindungen der Formel (VII) in der ersten Stufe mit einem Reduktionsmittel und in der zweiten Stufe in Gegenwart eines Kohlensäurederivates mit Verbindungen der Formel (III) oder in der zweiten Stufe mit Verbindungen der Formel (IV) umgesetzt werden.

Die Umsetzung erfogt wie in Verfahren [A] und [B] beschrieben.

Die Verbindungen der Formeln (III), (IV), (VI), (VIII) sind bekannt oder lassen sich nach bekannten Verfahren aus den entsprechenden Edukten synthetisieren.

### Syntheseschema 1:

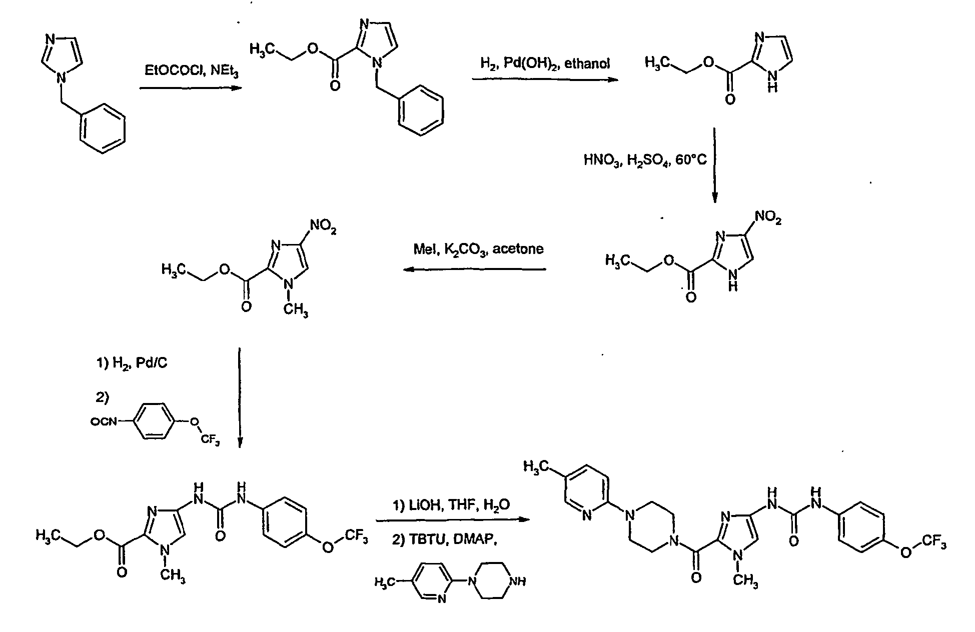

### Syntheseschema 2:

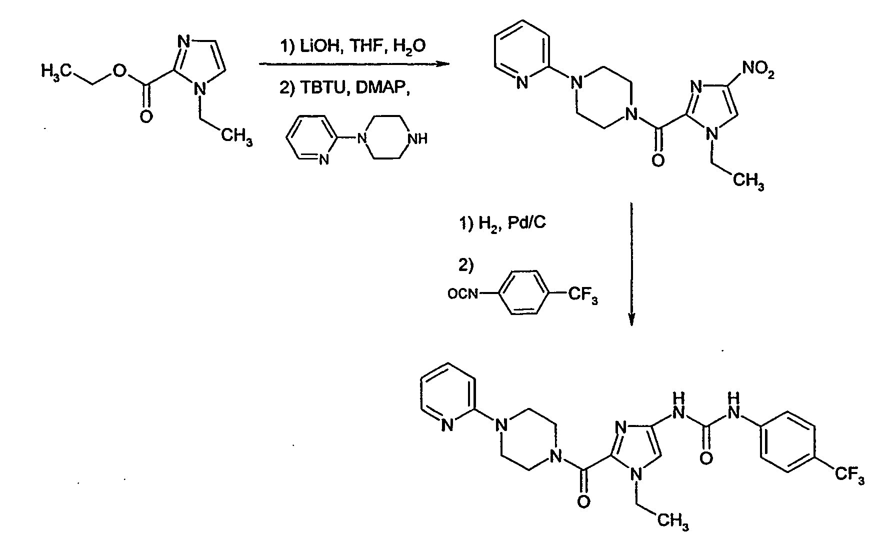

Die erfindungsgemäßen Verbindungen der allgemeinen Formel (I) zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegalieviren (CMV), insbesondere gegenüber dem humanen Cytomegalievirus (HCMV). Sie sind somit geeignet zur Behandlung und Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Die Verbindungen der allgemeinen Formel (I) können aufgrund ihrer besonderen Eigenschaften zur Herstellung von Arzneimitteln, die zur Prophylaxe und/oder Behandlung von Krankheiten, insbesondere Virusinfektionen, geeignet sind, verwendet werden.

Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und KrebsTherapie.
6) Behandlung von HCMV-positiven Krebspatienten mit dem Ziel, HCMV-vermittelte Tumorprogression zu verringern (vgl. J. Cinatl, et al., FEMS Microbiology Reviews 2004, 28, 59-77).

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalievirus, insbesondere dem humanen Cytomegalievirus, geeignet sind.

Die erfindungsgemäßen Verbindungen können aufgrund ihrer pharmakologischen Eigenschaften allein und bei Bedarf auch in Kombination mit anderen Wirkstoffen, insbesondere antiviralen Wirkstoffen wie beispielsweise Gancyclovir oder Acyclovir, zur Behandlung und/oder Prävention von Virusinfektionen, insbesondere von HCMV-Infektionen, eingesetzt werden.

Weiterer Gegenstand der Offenbarung ist der Einsatz der erfindungsgemäßen Verbindungen zur Behandlung und/oder Prophylaxe von Erkrankungen, vorzugsweise von Virusinfektionen, insbesondere von Infektionen mit dem humanen Cytomegalievirus (HCMV) oder einem anderen Vertreter der Gruppe der Herpes viridae.

Weiterer Gegenstand der Offenbarung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der Offenbarung ist ein Verfahren zur Behandlung und/oder Prophylaxe von Erkrankungen, insbesondere der zuvor genannten Erkrankungen, unter Verwendung einer antiviral wirksamen Menge der erfindungsgemäßen Verbindungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 25 mg/kg, vorzugsweise 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- BINAP: 2,2'-Bis-(diphenylphosphino)-1,1'-binaphthyl
- Bsp.: Beispiel
- CD₃CN: Deuteroacetonitril
- DC: Dünnschichtchromatographie
- DCI: direkte chemische Ionisation (bei MS)
- DCM: Dichlormethan
- DIEA: *N*,*N*-Diisopropylethylamin (Hünig Base)
- DMAP: 4-*N*,*N*-Dimethylaminopyridin
- DMF: *N*,*N*-Dimethylformamid
- DMSO: Dimethylsulfoxid
- d. Th.: der Theorie
- EDCI x HCl: N'-(3-Dimethylaminopropyl)-N-ethylcarbodiimid Hydrochlorid
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- h: Stunde
- HATU: *O*-(7-Azabenzotriazol-1-yl)-*N*,*N*,*N',N*'-tetramethyluroniumhexafluorophosphat
- HBTU: *O*-(Benzotriazol-1-yl)-*N,N,N',N'*-tetramethyluroniumhexafluorophosphat
- HPLC: Hochdruck-, Hochleistungsflüssigchromatographie
- i. V.: im Vakuum
- konz.: konzentriert
- LC-MS: Flüssigchromatographie-gekoppelte Massenspektroskopie
- LDA: Lithiumdiisopropylamid
- Lit.: Literatur(stelle)
- Lsg.: Lösung
- MS: Massenspektroskopie
- NMR: Kernresonanzspektroskopie
- proz.: prozentig
- RP-HPLC: Reverse Phase HPLC
- RT: Raumtemperatur
- Rₜ: Retentionszeit (bei HPLC)
- Schmp.: Schmelzpunkt
- TBTU: O-(Benzotriazol-1-yl)-N,N,N',N'-tetramethyluroniumtetrafluoroborat
- THF: Tetrahydrofuran
- verd.: verdünnt
- wässr.: wässrig

### HPLC- und LC-MS-Methoden:

**Methode 1 (LC-MS):** Instrument: Micromass Platform LCZ mit HPLC Agilent Serie 1100; Säule: Thermo HyPURITY Aquastar 3µ 50 mm x 2.1 mm; Eluent A: 1 L Wasser + 0.5 mL 50%ige Ameisensäure, Eluent B: 1 L Acetonitril + 0.5 mL 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 mL/min; UV-Detektion: 210 nm.

**Methode 2 (LC-MS):** Instrument: Micromass Quattro LCZ mit HPLC Agilent Serie 1100; Säule: Phenomenex Synergi 2µ Hydro-RP Mercury 20 mm x 4 mm; Eluent A: 1 L Wasser + 0.5 mL 50%ige Ameisensäure, Eluent B: 1 L Acetonitril + 0.5 mL 50%ige Ameisensäure; Gradient: 0.0 min 90%A → 2.5 min 30%A → 3.0 min 5%A → 4.5 min 5%A; Fluss: 0.0 min 1 mL/min, 2.5 min/3.0 min/4.5 min 2 mL/min; Ofen: 50°C; UV-Detektion: 208- 400 nm.

**Methode 3 (LC-MS):** Instrument: Micromass Platform LCZ mit HPLC Agilent Serie 1100; Säule: Phenomenex Synergi 2µ Hydro-RP Mercury 20 mm x 4 mm; Eluent A: 1 L Wasser + 0.5 mL 50%ige Ameisensäure, Eluent B: 1 L Acetonitril + 0.5 mL 50%ige Ameisensäure; Gradient: 0.0 min 90%A → 2.5 min 30%A → 3.0 min 5%A → 4.5 min 5%A; Fluss: 0.0 min 1 mL/min, 2.5 min/3.0 min/4.5 min 2 mL/min; Ofen: 50°C; UV-Detektion: 210 nm.

**Methode 4 (LC-MS)-** Gerätetyp MS: Micromass ZQ; Gerätetyp HPLC: Waters Alliance 2795; Säule: Phenomenex Synergi 2µ Hydro-RP Mercury 20 mm x 4mm; Eluent A: 1 L Wasser + 0.5 mL 50%ige Ameisensäure, Eluent B: 1 L Acetonitril + 0.5 mL 50%ige Ameisensäure; Gradient: 0.0 min 90%A → 2.5 min 30%A → 3.0 min 5%A → 4.5 min 5%A; Fluss: 0.0 min 1 mL/min, 2.5 min/3.0 min/4.5 min 2 mL/min; Ofen: 50°C; UV-Detektion: 210 nm.

**Methode 5 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 L Wasser + 0.5 mL 50%ige Ameisensäure, Eluent B: 1 L Acetonitril + 0.5 mL 50%ige Ameisensäure; Gradient: 0.0 min 90%A → 2.5 min 30%A → 3.0 min 5%A → 4.5 min 5%A; Fluss: 0.0 min 1 mL/min, 2.5 min/3.0 min/4.5 min. 2 mL/min; Ofen: 50°C; UV-Detektion: 210 nm.

**Methode 6 (LC-MS):** Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µl 50%ige Ameisensäure /1, Eluent B: Acetonitril + 500 µl 50%ige Ameisensäure /1; Gradient: 0.0 min 0%B → 2.9 min 70%B → 3.1 min 90%B → 4.5 min 90%B; Ofen: 50°C; Fluss: 0.8 mL/min; UV-Detektion: 210 nm.

**Methode 7 (LC-MS):** Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%ige Ameisensäure / 1; Eluent B: Acetonitril + 500 µl 50%ige Ameisensäure / 1; Gradient: 0.0 min 10%B → 3.0 min 95%B → 4.0 min 95%B; Ofen: 35°C; Fluss: 0.0 min 1.0 mL/min → 3.0 min 3.0 mL/min → 4.0 min 3.0 mL/min; UV-Detektion: 210 nm.

**Methode 8 (LC-MS):** Instrument: Micromass Quattro LCZ, mit HPLC Agilent Serie 1100; Säule: Grom-SIL120 ODS-4 HE, 50 mm x 2.0 mm, 3 µm; Eluent A: 1 L Wasser + 1 mL 50%ige Ameisensäure, Eluent B: 1L Acetonitril + 1 mL 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 mL/min; UV-Detektion: 208-400 nm.

**Methode 9 (GC-MS):** Instrument: Micromass GCT, GC6890; Säule: Restek RTX-35MS, 30 m x 250 µm x 0.25 µm; konstanter Fluss mit Helium: 0.88 mL/min; Ofen: 60°C; Inlet: 250°C; Gradient: 60°C (0.30 min halten), 50°C/min → 120°C, 16°C/min → 250°C, 30°C/min → 300°C (1.7 min halten). **Methode 10 (analytische HPLC):** Säule: Kromasil 100 RP-18, 60 mm x 2.1 mm, 3.5 µm; Eluent A: Wasser + 0.5% Perchlorsäure (70 %ig), Eluent B: Acetonitril; Gradient: 0 min 2%B, 0.5 min 2%B, 4.5 min 90%B, 9 min 90%B, 9.2 min 2%B, 10 min 2%B; Fluss: 0.75 mL/min; Säulentemperatur: 30°C; Detektion: UV 210 nm.

### Ausgangsverbindungen

### Beispiel 1A

### 1-Benzyl-1H-imidazol-2-carbonsäureethylester

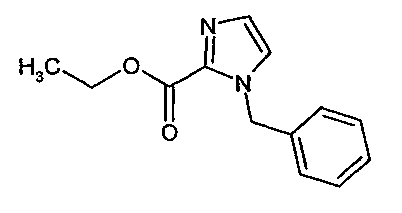

148 g (936 mmol) 1-Benzyl-1H-imidazol werden in 480 mL Acetonitril suspendiert und bei 20°C mit 120 mL (87.1 g; 860 mmol) Triethylamin versetzt. Innerhalb von 15 Minuten werden dann 211.2 mL (239 g; 2208 mmol) Chlorameisensäureethylester zugetropft. Die Reaktionsmischung wird 10 Minuten bei -20°C gerührt. Nach Erwärmen auf 15 bis 20°C wird die Reaktionsmischung 18 h gerührt und dann im Vakuum eingeengt. Der Rückstand wird mit Wasser, gesättigter Natriumchloridlösung und gesättigter Natriumhydrogencarbonatlösung versetzt und dreimal mit Ethylacetat extrahiert. Die vereinigten organischen Phasen werden mit gesättigter Natriumchloridlösung gewaschen und nach Trocknen mit Magnesiumsulfat im Vakuum eingedampft. Der Rückstand wird im Hochvakuum fraktioniert destilliert (Siedepunkt = 173 bis 181°C, Druck = 1.7 bis 1.2 mbar).
Ausbeute: 122.6 g (46 % d. Th.)
LC-MS (Methode 4): Rₜ = 1.71 min.
MS (ESI⁺): m/z = 231 [M+H]⁺
¹H-NMR (300MHz, DMSO-d₆): δ = 7.6 (s, 1H), 7.4 - 7.1 (m, 6H), 5.2 (s, 2H), 4.25 (q, 2H), 1.25 (tr, 3H) ppm.

### Beispiel 2A

### Imidazol-2-carbonsäureethylester

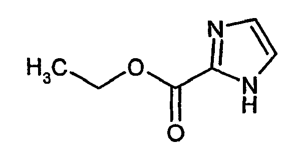

34.7 g (150.9 mmol) 1-Benzyl-1H-imidazol-2-carbonsäureethylester werden in 1005 mL Ethanol gelöst und mit 34 g Ammoniumformiat versetzt. Die Reaktionsmischung wird ca. 6 h zum Rückfluss erhitzt. Dabei werden in kleinen Portionen insgesamt 8 g 10% Palladium auf Aktivkohle und 18 g Ammoniumformiat zugegeben. Nach Abkühlen wird der Katalysator abfiltriert und das Filtrat im Vakuum eingeengt. Das dabei auskristallisierende Produkt wird in 80 mL Eiswasser verrührt und abgesaugt.
Ausbeute: 15.9 g (75 % d. Th.)
MS (ESI⁺): m/z =141 [M+H]⁺
¹H-NMR (200MHz, DMSO-d₆): δ = 13.3 (s breit, 1H), 7.4 (s, 1H), 7.15 (s, 1H), 4.3 (q, 2H), 1.3 (tr, 3H) ppm.

### Beispiel 3A

### 4-Nitro-1H-imidazol-2-carbonsäureethylester

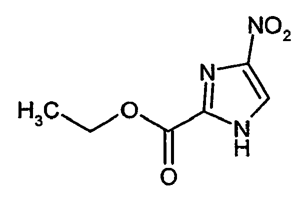

16.08 g (114.7 mmol) Imidazol-2-carbonsäureethylester werden unter Eiskühlung in 71.7 mL konzentrierter Schwefelsäure gelöst. Dann werden 71.7 mL 100%ige rauchende Salpetersäure zugetropft. Die Reaktionslösung wird 3 h bei 50 bis 60°C gerührt und nach Abkühlen auf 800 mL Eis/Wasser-Gemisch gegossen. Die ausfallenden Kristalle werden abgesaugt und mit 1500 mL Eiswasser gewaschen.
Ausbeute: 15 g (70 % d. Th.)
MS (ESI⁺): m/z = 186 [M+H]⁺
¹H-NMR (300MHz, DMSO-d₆): δ = 14.5 (s breit, 1H), 8.5 (s, 1H), 4.4 (q, 2H), 1.35 (tr, 3H), ppm.

### Beispiel 4A

### 4-({[(4-Chlor-2-methylphenyl)amino]carbonyl}amino)-1-(cyclopropylmethyl)-1H-imidazol-2-carbonsäure

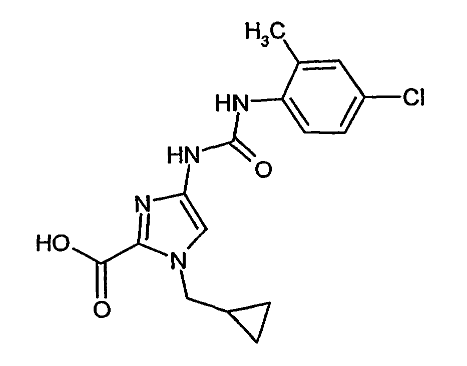

### Stufe 1

### 1-(Cyclopropylmethyl)-4-nitro-1H-imidazol-2-carbonsäureethylester

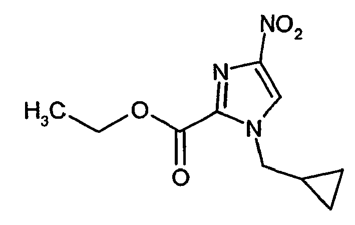

15 g (81 mmol) 4-Nitro-1H-imidazol-2-carbonsäureethylester werden unter Argon zusammen mit 13.13 g (97.2 mmol) Cyclopropylmethylbromid und 22.4 g (162 mmol) Kaliumcarbonat in 165 mL DMF 1 h bei 80°C gerührt. Nach Abkühlen wird die Reaktionsmischung mit Wasser verdünnt und viermal mit Ethylacetat extrahiert. Die vereinigten organischen Phasen werden einmal mit Wasser und dreimal mit gesättigter Natriumchloridlösung gewaschen, mit Magnesiumsulfat getrocknet und im Vakuum eingedampft. Der kristalline Rückstand wird für die nächste Reaktion direkt weiterverwendet.
Ausbeute: 17.59 g (70 % d. Th.)
LC-MS (Methode 2): Rₜ = 2.02 min.
MS (ESI⁺): m/z = 240 [M+H]⁺
¹H-NMR (300MHz, DMSO-d₆): δ = 8.2 (s, 1H), 4.4 (q, 2H), 4.3 (d, 2H), 1.4 (m, 4H), 0.55 (q, 2H), 0.45 (q, 2H) ppm.

### Stufe 2

### 4-Amino-1-(cyclopropylmethyl)-1H-imidazol-2-carbonsäureethylester

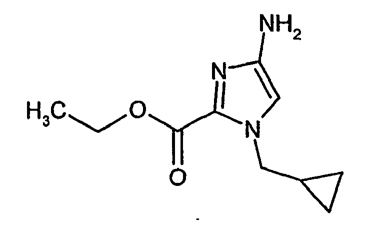

3.89 g (16.26 mmol) 1-(Cyclopropylmethyl)-4-nitro-1H-imidazol-2-carbonsäureethylester werden in 50 mL THF gelöst und mit einer Spatelspitze Raney-Nickel versetzt. Die Reaktionsmischung wird in einer Hydrierungsapparatur bei Raumtemperatur mit Wasserstoff hydriert. Der Katalysator wird abfiltriert und das Filtrat im Vakuum eingedampft. Der Eindampfrückstand wird für die nächste Reaktion direkt weiterverwendet.
Ausbeute: 3.46 g (100 % d. Th.)
LC-MS (Methode 3): Rₜ = 1.21 min.
MS (ESI⁺): m/z = 210 [M+H]⁺
¹H-NMR (300MHz, DMSO-d₆): δ = 6.55 (s, 1H), 4.55 (s, 2H), 4.2 (q, 2H), 4.1 (d, 2H), 1.25 (tr, 3H), 1.2 (m, 1H), 0.5 (q, 2H), 0.3 (q, 2H) ppm.

### Stufe 3

### 4-({[(4-Chlor-2-methylphenyl)amino]carbonyl}amino)-1-(cyclopropylmethyl)-1H-imidazol-2-carbonsäureethylester

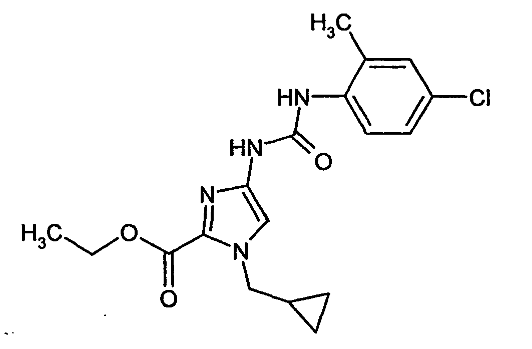

7.49 g (35.8 mmol) 4-Amino-1-(cyclopropylmethyl)-1H-imidazol-2-carbonsäureethylester werden in 18 mL THF unter Argon mit 6 g (35.8 mmol) 3-Chlor-4-phenylisocyanat versetzt und 4 h bei Raumtemperatur gerührt. Die Reaktionsmischung wird im Vakuum eingeengt und das dabei auskristallisierende Produkt in 40 mL Ethylacetat verrührt und abgesaugt.
Ausbeute: 11.1 g (82 % d. Th.)
LC-MS (Methode 2): Rₜ = 2.66 min.
MS (ESI⁺): m/z= 376 [M+H]⁺
¹H-NMR (300MHz, DMSO-d₆): δ = 9.45 (s, 1H), 8.0 (d, 1H), 7.35 (s, 1H), 7.3 (d, 1H), 7.2 (dd, 1H), 4.3 (q, 2H), 4.25 (d, 2H), 2.25 (s, 3H), 1.3 (tr, 3H), 1.25 (m, 1H), 0.55 (q, 2H), 0.35 (q, 2H) ppm.

### Stufe 4

### 4-({[(4-Chlor-2-methylphenyl)amino]carbonyl}amino)-1-(cyclopropylmethyl)-1H-imidazol-2-carbonsäure

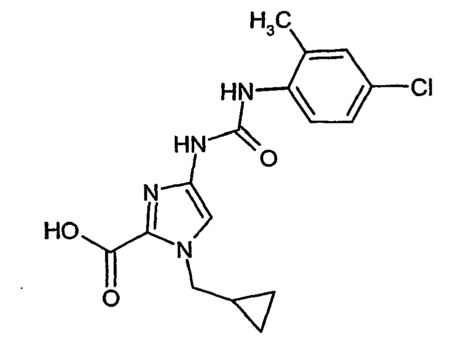

10.6 g (28.1 mmol) 4-({[(4-Chlor-2-methylphenyl)amino]carbonyl}amino)-1-(cyclopropylmethyl)-1H-imidazol-2-carbonsäureethylester werden in 158 mL Ethanol suspendiert. Unter Eiskühlung werden 16.4 mL Wasser und 6 mL (112 mmol) 50%-ige wässrige Natronlauge-Lösung zugegeben. Die Reaktionsmischung wird 1 h bei Raumtemperatur gerührt und dann im Vakuum eingeengt. Der Rückstand wird in 100 mL Isopropanol aufgenommen und unter Eiskühlung mit 100 mL 1N Salzsäure versetzt. Die Kristalle werden abgesaugt und im Vakuum bei 40°C getrocknet.
Ausbeute: 9.85 g (100 % d. Th.)
LC-MS (Methode 4): Rₜ = 1.74 min.
MS (ESI⁺): m/z = 349 [M+H]⁺
¹H-NMR (400MHz, DMSO-d₆): δ = 9.4 (s, 1H), 8.0 (d, 1H), 7.3 (s, 1H), 7.25 (d, 1H), 7.2 (dd, 1H), 4.25 (d, 2H), 2.25 (s, 3H), 1.25 (m, 1H), 0.55 (q, 2H), 0.35 (q, 2H) ppm.

### Beispiel 5A

### 1-(Cyclopropylmethyl)-4-[({[4-(trifluormethoxy)phenyl]amino}carbonyl)amino]-1H-imidazol-2-carbonsäure

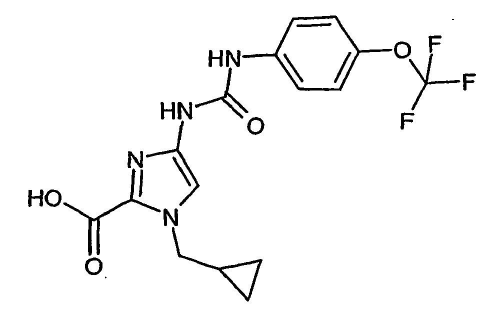

Die Herstellung erfolgt analog zu Beispiel 4A.
Ausbeute: 10.2 g (93 % d. Th.)
LC-MS (Methode 4): Rₜ = 1.87 min.
MS (ESI⁺): m/z = 3 85 [M+H]⁺
¹H-NMR (400MHz, DMSO-d₆): δ = 8.6 (s, 1H), 8.4 (s, 1H), 7.55 (d, 2H), 7.4 (s, 1H), 7.25 (d, 2H), 4.25 (d, 2H), 1.25 (m, 1H), 0.55 (q, 2H), 0.35 (q, 2H) ppm.

### Beispiel 6A

### 1-Butyl-4-({[(4-chlor-2-methylphenyl)amino]carbonyl}amino)-1H-imidazol-2-carbonsäure

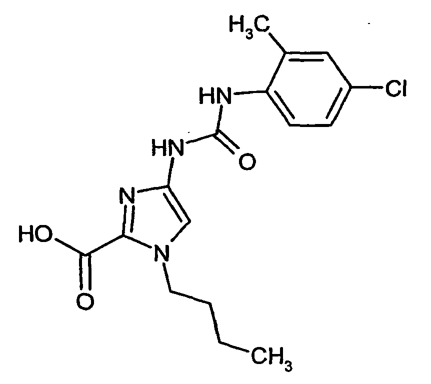

Die Herstellung erfolgt analog zu Beispiel 4A.
Ausbeute: 2.2 g (93 % d. Th.)
LC-MS (Methode 4): Rₜ = 1.83 min.
MS (ESI⁺): m/z = 351 [M+H]⁺
¹H-NMR (300MHz, DMSO-d₆): δ = 9.35 (s, 1H), 8.0 (d, 1H), 7.3 (s, 1H), 7.25 (d, 1H), 7.2 (dd, 1H), 4.35 (tr, 2H), 2.25 (s, 3H), 1.7 (quintett, 2H), 1.25 (sextett, 2H), 0.9 (tr, 3H) ppm.

### Beispiel 7A

### 1-Butyl-4-[({[4-(trifluormethoxy)phenyl]amino}carbonyl)amino]-1H-imidazol-2-carbonsäure

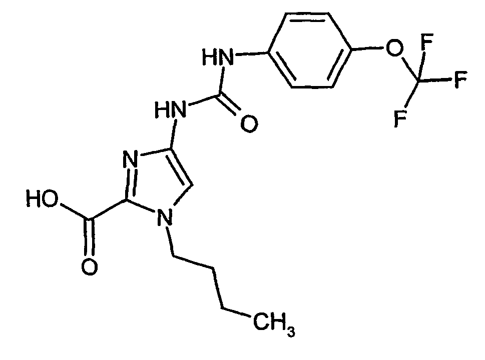

Die Herstellung erfolgt analog zu Beispiel 4A.
Ausbeute: 2.05 g (96 % d. Th.)
LC-MS (Methode 4): Rₜ = 1.96 min.
MS (ESI⁺): m/z = 387 [M+H]⁺
¹H-NMR (300MHz, DMSO-d₆): δ = 9.0 (s, 1H), 8.9 (s, 1H), 7.55 (d, 2H), 7.3 (s, 1H), 7.25 (d, 1H), 4.35 (tr, 2H), 1.7 (quintett, 2H), 1.25 (sextett, 2H), 0.9 (tr, 3H) ppm.

### Beispiel 8A

### 4-[({[4-(Trifluormethoxy)phenyl]amino}carbonyl)amino]-1-[4-(trifluormethyl)benzyl]-1H-imidazol-2-carbonsäure

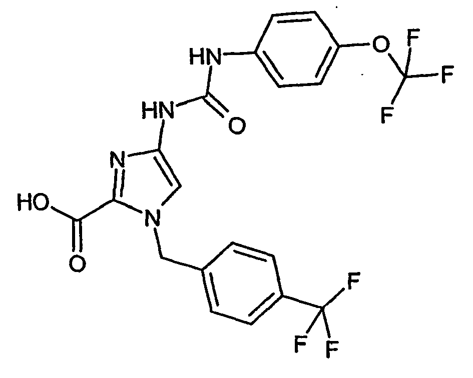

Herstellung analog zu Beispiel 4A.
Ausbeute: 15.2 g (100 % d. Th.)
LC-MS (Methode 2): Rₜ = 2.46 min.
MS (ESI⁺): m/z = 489 [M+H]⁺
¹H-NMR (300MHz, DMSO-d₆): δ = 9.15 (s, 1H), 9.05 (s, 1H), 7.75 (d, 2H), 7.55 (d, 2H), 7.45 (s, 1H), 7.35 (d, 2H), 7.25 (d, 2H), 5.7 (s, 2H) ppm.

### Beispiel 9A

### 4-({[(4-Chlor-2-methylphenyl)amino]carbonyl}amino)-1-[4-(trifluormethyl)benzyl]-1H-imidazol-2-carbonsäure

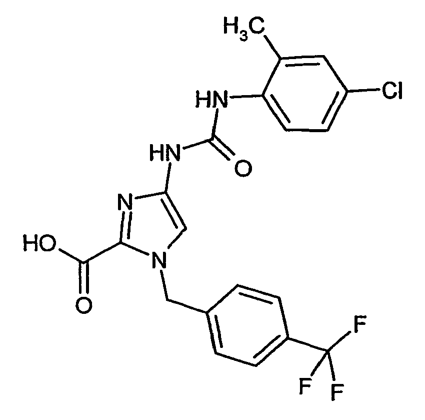

Herstellung analog zu Beispiel 4A.
Ausbeute: 15.6 g (100 % d. Th.)
LC-MS (Methode 4): Rₜ = 2.23 min.
MS (ESI⁺): m/z = 453 [M+H]⁺
¹H-NMR (300MHz, DMSO-d₆): δ = 9.5 (s, 1H), 7.95 (d, 1H), 7.75 (d, 2H), 7.4 (d, 2H), 7.35 (s, 1H), 7.25 (s, 1H), 7.15 (d, 1H), 5.75 (s, 2H), 2.25 (s, 3H) ppm.

### Beispiel 10A

### 1-Methyl-4-nitro-1H-imidazol-2-carbonsäureethylester

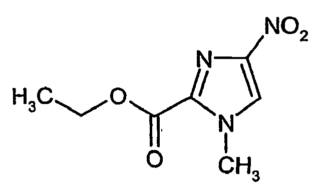

6.80 g (36.7 mmol) 4-Nitro-1*H*-imidazol-2-carbonsäureethylester werden in 140 mL Aceton gelöst und mit 11.2 g (80.8 mmol) Kaliumcarbonat sowie 4.57 mL (73.5 mmol) Iodmethan versetzt. Anschließend lässt man 4 h bei 60°C rühren. Laut DC (Cyclohexan/Ethylacetat 2:1) hat sich das Edukt vollständig umgesetzt. Nach dem Abkühlen wird filtriert, der Rückstand mit Dichlormethan gewaschen und das Filtrat vom Lösungsmittel befreit. Der erhaltene Feststoff wird im Vakuum getrocknet.
Ausbeute: 7.0 g (95 % d. Th.)
LC-MS (Methode 5): Rₜ = 1.40 min.
MS (ESI⁺): m/z = 200 [M+H]⁺
¹H-NMR (400MHz, DMSO-d₆): δ = 8.64 (s, 1H), 4.35 (q, 2H), 3.99 (s, 3H), 1.34 (t, 3H).

### Beispiel 11A

### 4-Amino-1-methyl-1H-imidazol-2-carbonsäureethylester

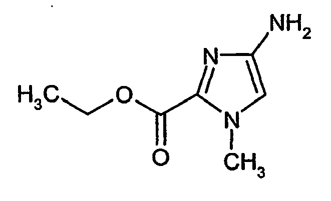

0.50 g (2.5 mmol) 1-Methyl-4-nitro-1*H*-imidazol-2-carbonsäureethylester werden in 7.5 mL Ethanol gelöst, mit 0.13 g (0.13 mmol) Palladium auf Aktivkohle (10 %ig) versetzt und 12 h bei 3 bar hydriert. Anschließend wird die Reaktionslösung über Kieselgur filtriert und das Filtrat eingeengt. Der Rückstand wird im Vakuum getrocknet und ohne weitere Reinigung weiter umgesetzt.
Ausbeute: 0.42 g (99 % d. Th.)
LC-MS (Methode 1): Rₜ = 1.59 min.
MS (ESI⁺): m/z = 170 [M+H]⁺
¹H-NMR (400MHz, DMSO-d₆): δ = 6.47 (s, 1H), 4.55 (bs, 2H), 4.19 (q, 2H), 3.80 (s, 3H), 1.28 (t, 3H).

### Beispiel 12A

### 1-Methyl-4-[({[4-(trifluormethoxy)phenyl]amino}carbonyl)amino]-1H-imidazol-2-carbonsäure-ethylester

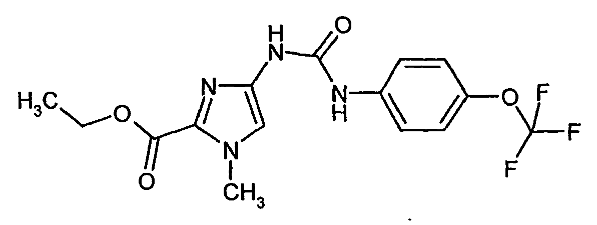

1.22 g (3.61 mmol) 4-Amino-1-methyl-1*H*-imidazol-2-carbonsäureethylester (Synthese analog Beispiel 4A Stufe 3 oder auch gemäß Tetrahedron Lett. 2003, 44, 1607 und dort zitierter Literatur) werden in 50 mL THF unter Argon mit 1.46 g (7.21 mmol) 4-(Trifluormethoxy)phenylisocyanat versetzt und über Nacht bei Raumtemperatur gerührt. Die Reaktionsmischung wird filtriert, das Filtrat im Vakuum eingeengt und chromatographisch gereinigt.
Ausbeute: 860 mg (62 % d. Th.)
LC-MS (Methode 5): R₁ = 2.41 min.
MS (ESI⁺): m/z = 373 [M+H]⁺
¹H-NMR (300MHz, DMSO-d₆): δ = 8.98 (bs, 2H), 7.55 (m, 2H), 7.36 (s, 1H), 7.29 (m, 2H), 4.28 (q, 2H), 3.91 (s, 3H), 1.30 (t, 3H).

### Beispiel 13A

### 1-Methyl-4-[({[4-(trifluormethoxy)phenyl]amino}carbonyl)amino]-1H-imidazol-2-carbonsäure

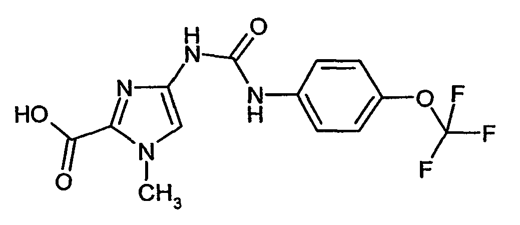

835 mg (2.13 mmol) 1-Methyl-4-[({[4-(trifluormethoxy)phenyl]amino}carbonyl)amino]-1H-imidazol-2-carbonsäureethylester werden in 5 mL Ethanol und 12 mL Tetrahydrofuran suspendiert. Unter Eiskühlung werden 2 mL (25 mmol) 50%ige wässrige Natronlauge-Lösung zugegeben. Die Reaktionsmischung wird über Nacht bei Raumtemperatur gerührt und dann unter Eiskühlung mit 1N Salzsäure sauer gestellt. Die Lösung wird mit Dichlormethan extrahiert. Die organische Phase wird im Vakuum eingeengt. Der Rückstand wird durch präparative HPLC gereinigt.
Ausbeute: 346 mg (44 % d. Th.).
LC-MS (Methode 4): Rₜ = 1.62 min.
MS (ESI⁺): m/z = 345 [M+H]⁺
¹H-NMR (400MHz, DMSO-d₆): δ = 9.33 (bs, 1H), 8.98 (bs, 1H), 7.55 (m, 2H), 7.30 (s, 1H), 7.28 (m, 2H), 3.90 (s, 3H),

### Beispiel 14A

### 1-(5-Methylpyridin-2-yl)piperazin

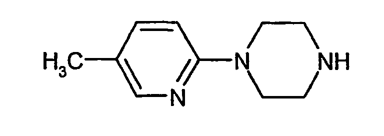

### Stufe 1

### 1-(tert-Butyloacycarbonyl)-4-(5-methylpyridin-2-yl)piperazin

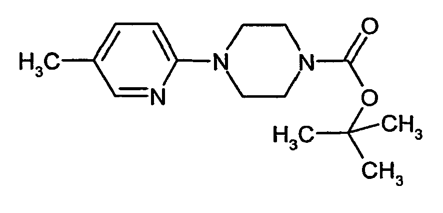

Unter einer Argonatmosphäre werden 2.50 g (19.6 mmol) 2-Methyl-5-chlorpyridin und 4.38 g (23.5 mmol) N-(tert-Butyloxycarbonyl)-piperazin in 50 mL absolutem Toluol gelöst. Anschließend gibt man 2.26 g (23.5 mmol) Natrium-tert-butylat, 0.37 g (0.59 mmol) BINAP und 0.36 g (0.39 mmol) Tris(dibenzylidenaceton)dipalladium hinzu und erhitzt 12 h auf 70°C. Nach dem Abkühlen wird das Reaktionsgemisch mit Diethylether versetzt, dreimal mit gesättigter Natriumchloridlösung gewaschen, über Natriumsulfat getrocknet und im Vakuum vom Lösungsmittel befreit. Der Rückstand wurde flashchromatographisch (Cyclohexan/Ethylacetat 9:1) gereinigt.
Ausbeute: 5.27 g (97 % d. Th.).
LC-MS (Methode 4): Rₜ = 1.26 min.
MS (ESI⁺): m/z = 278 [M+H]⁺
¹H-NMR (300MHz, CDCl₃): δ = 8.02 (d, 1H), 7.34 (dd, 1H), 6.59 (d, 1H), 3.55 (m, 4H), 3.45 (m, 4H), 2.21 (s, 3H), 1.49 (s, 9H).

### Stufe 2

### 1-(5-Methylpyridin-2-yl)piperazin

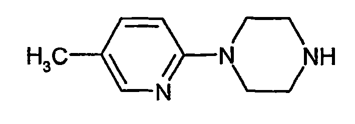

3.47 g (12.5 mmol) 1-(tert-Butyloxycarbonyl)-4-(5-methylpyridin-2-yl)piperazin werden in 10 mL Dioxan gelöst und mit 31 mL (125 mmol) Chlorwasserstoff in Dioxan (4 molar) versetzt. Man lässt 2 h bei RT rühren. Anschließend wird eingeengt, der Rückstand mit 1M Natronlauge alkalisiert und mehrmals mit Dichlormethan extrahiert. Die vereinigten organischen Phasen werden über Natriumsulfat getrocknet, eingeengt und im Vakuum getrocknet.
Ausbeute: 2.18 g (98 % d. Th.).
LC-MS (Methode 5): Rₜ = 0.38 min.
MS (ESI⁺): m/z = 177 [M+H]⁺
¹H-NMR (300MHz, CDCl₃): δ = 8.02 (d, 1H), 7.32 (dd, 1H), 6.59 (d, 1H), 3.45 (m, 4H), 3.00 (m, 4H), 2.20 (s, 3H).

### Beispiel 15A

### 1-Ethyl-4-[({[4-(trifluormethoxy)phenyl]amino}carbonyl)amino]-1H-imidazol-2-carbonsäure

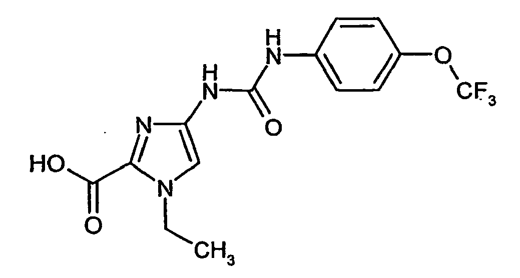

Die Herstellung erfolgt analog zu Beispiel 13A.
Ausbeute: 425 mg (91 % d. Th.).
LC-MS (Methode 5): Rₜ = 1.94 min.
MS (ESI⁺): m/z = 359 [M+H]⁺
¹H-NMR (300MHz, DMSO-d₆): δ = 10.3 (bs, 1H), 7.67 (m, 2H), 7.24 (s, 1H), 7.20 (m, 2H), 4.45 (q, 2H), 1.33 (t, 3H).

### Beispiel 16A

### 1-Butyl-4-[({[4-(trifluormethyl)phenyl]amino}carbonyl)amino]-1H-imidazol-2-carbonsäure

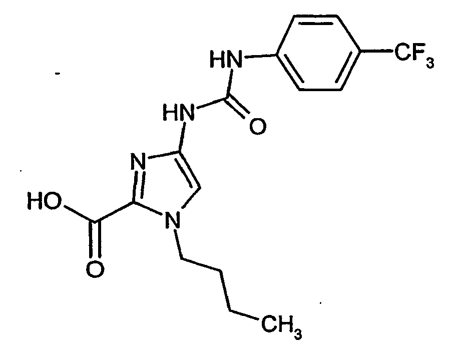

Die Herstellung erfolgt analog zu Beispiel 13A.
Ausbeute: 1.71 g (90 % d. Th.)
LC-MS (Methode 2): Rₜ = 2.13 min.
MS (ESI⁺): m/z = 371 [M+H]⁺
¹H-NMR (300MHz, DMSO-d₆): δ = 9.30 (bs, 1H), 9.03 (bs, 1H), 7.64 (m, 4H), 7.36 (s, 1H), 4.35 (t, 2H), 1.68 (quint, 2H), 1.26 (sext, 2H), 0.89 (t, 3H).

### Beispiel 17A

### 1-(5-Fluorpyridin-2-yl)piperazin

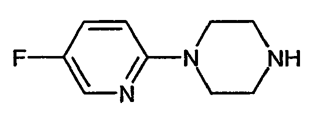

500 mg (2.84 mmol) 2-Brom-5-fluorpyridin und 1.22 g (14.2 mmol) Piperazin werden unter Rühren 24 h auf 150°C erhitzt. Nach dem Abkühlen wird überschüssiges Piperazin unter vermindertem Druck abdestilliert (Kugelrohr, 1.5 mbar, 120°C). Der Rückstand wird durch Flashchromatographie (Dichlormethan/Ethanol/konz. Ammoniaklösung, 30:1:0.1) gereinigt.
Ausbeute: 267 mg (52 % d. Th.).
LC-MS (Methode 9): Rₜ = 8.07 min.
MS (DCI): m/z = 182 [M+H]⁺
¹H-NMR (400MHz, DMSO-d₆): δ = 8.07 (d, 1H), 7.48 (td, 1H), 6.82 (dd, 1H), 3.32 (t, 4H), 2.78 (t, 4H).

### Bespiel 18A

### 1-(5-Brompyridin-2-yl)piperazin

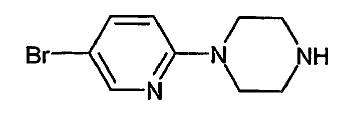

Die Herstellung erfolgt analog zu Beispiel 17A.
Ausbeute: 827 mg (81 % d. Th.).
LC-MS (Methode 1): Rₜ = 2.02 min.
MS (ESI⁺): m/z = 242 [M+H]⁺
¹H-NMR (300MHz, DMSO-d₆): δ = 8.15 (d, 1H), 7.65 (dd, 1H), 6.79 (d, 1H), 3.38 (m, 4H), 2.74 (m, 4H).

### Beispiel 19A

### 1-(5-Methoxypyridin-2-yl)piperazin

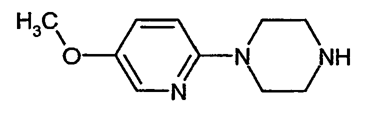

Die Herstellung erfolgt analog zu Beispiel 14A.
Ausbeute: 91 mg (90 % d. Th.).
¹H-NMR (400MHz, CDCl₃): δ = 7.94 (d, 1H), 7.15 (dd, 1H), 6.64 (d, 1H), 3.80 (s, 3H), 3.48 (m, 4H), 3.00 (m, 4H).

### Beispiel 20A

### 4-[({[4-(Difluormethoxy)phenyl]amino}carbonyl)amino]-1-methyl-1H-imidazol-2-carbonsäure

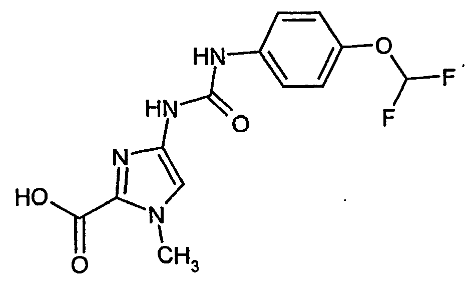

Die Herstellung erfolgt analog zu Beispiel 13A.
Ausbeute: 964 mg (81 % d. Th.).
HPLC (Methode 10): Rₜ = 3.57 min.
MS (ESI⁺): m/z = 327 [M+H]⁺
¹H-NMR (400MHz, CDCl₃): δ = 8.9 (s, 1H), 8.8 (s, 1H), 7.5 (d, 2H), 7.3 (s, 2H), 7.1 (t, 1H), 7.09 (d, 2H), 3.9 (s, 3H).

### Beispiel 21A

### 1-[(1-Ethyl-4-nitro-1H-imidazol-2-yl)carbonyl]-4-(pyridin-2-yl)piperazin

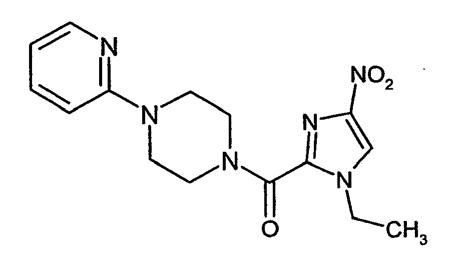

Eine Mischung von 1.23 g (5.06 mmol) 1-Ethyl-4-nitro-1H-imidazole-2-carbonsäureethylester (hergestellt analog Beispiel 10A) und 2.48 g (15.2 mmol) N-(Pyridin-2-yl)piperazin wird über Nacht bei 100°C gerührt. Zur Aufarbeitung wird das erhaltene Rohgemisch über präparative HPLC gereinigt. Man erhält 0.724 g (43 % d.Th.) des Produktes.
HPLC (Methode 10): Rₜ = 3.19 min.
MS (ESI⁺): m/z = 331 [M+H]⁺
¹H-NMR (300MHz, DMSO-d₆): δ = 8.7 (s, 1H), 8.1 (m, 1H), 7.55 (m, 1H), 6.9 (d, 1H), 6.65 (dd, 1H), 4.2 (q, 2H), 3.8 (m, 4H), 3.65 (m, 2H), 3.55 (m, 2H), 1.4 (t, 3H).

### Beispiel 22A

### 4-[({[4-(Difluormethoxy)phenyl]amino}carbonyl)amino]-1-butyl-1H-imidazol-2-carbonsäure

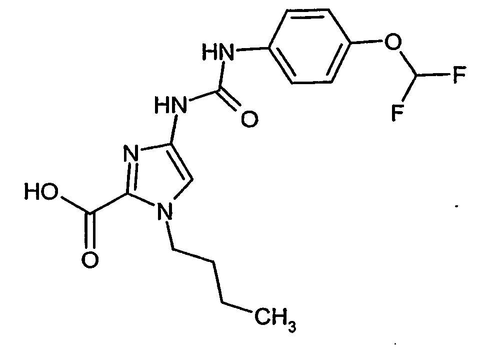

Die Herstellung erfolgt analog zu Beispiel 13A.
Ausbeute: 1.06 g (71 % d. Th.).
HPLC (Methode 10): Rₜ = 4.046 min.
MS (ESI⁺): m/z = 369 [M+H]⁺
¹H-NMR (400MHz, CDCl₃): δ = 11.1 (s, 1H), 7.7 (d, 2H), 7.1 (t, 1H), 7.05 (m, 3H), 4.5 (t, 2H), 1.7 (m, 2H), 1.3 (m, 2H), 0.9 (t, 3H).

### Beispiel 23A

### 1-[(1-Methyl-4-nitro-1H-imidazol-2-yl)carbonyl]-4-(pyridin-2-yl)piperazin

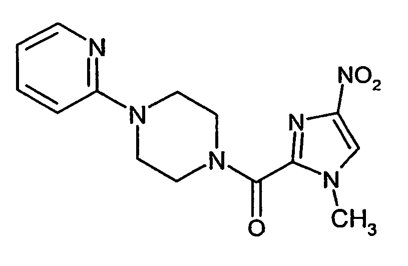

Die Herstellung erfolgt analog zu Beispiel 21A.
Ausbeute: 4 g (72 % d. Th.)
HPLC (Methode 10): Rₜ = 2.99 min.
MS (ESI⁺): m/z = 317 [M+H]⁺
¹H-NMR (300MHz, DMSO-d₆): δ = 8.6 (s, 1H), 8.15 (m, 1H), 7.55 (m, 1H), 6.9 (d, 1H), 6.7 (dd, 1H), 3.9 (m, 5H), 3.8 (m, 2H), 3.7-3.5 (m, 4H).

### Beispiel 24A

### 1-Methyl-4-[({[4-(trifluormethyl)phenyl]amino}carbonyl)amino]-1H-imidazol-2-carbonsäure

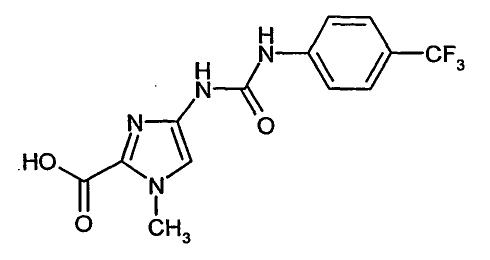

Die Herstellung erfolgt analog zu Beispiel 13A.
Ausbeute: 168 mg (99 % d. Th.).
HPLC (Methode 4): Rₜ = 1.57 min.
MS (ESI⁺): m/z = 329 [M+H]⁺
¹H-NMR (400MHz, CDCl₃): δ = 9.80 (bs, 1H), 9.18 (bs, 1H), 7.65 (m, 4H), 7.48 (s, 1H), 3.92 (s, 3H).

### Ausführungsbeispiele

### Beispiel 1

### N-{1-Methyl-2-[(4-pyridin-2-yl-piperazin-1-yl)carbonyl]-1H-imidazol-4-yl}-N'-[4-(trifluormethoxy)phenyl]harnstoff

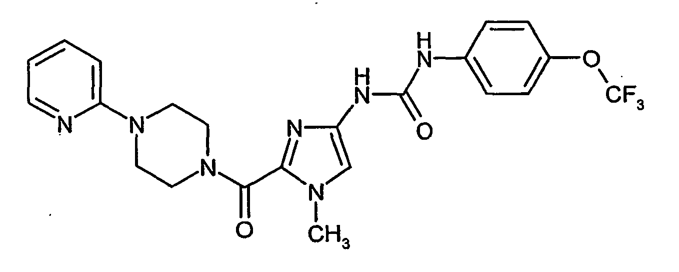

1.50 g (4.36 mmol) Beispiel 13A werden in 30 mL DMF gelöst und mit 1.82 g (5.66 mmol) O-(Benzotriazol-1-yl)-N,N,N',N'-tetramethyluroniumtetrafluoroborat (TBTU) und 266 mg (2.18 mmol) 4-Dimethylaminopyridin versetzt. Nach Zugabe von 925 mg (5.66 mmol) 1-(Pyridin-2-yl)-piperazin lässt man 4 h bei RT rühren. Das Reaktionsgemisch wird durch RP-HPLC gereinigt.
Ausbeute: 1.79 g (83 % d. Th.).
LC-MS (Methode 2): Rₜ = 1.83 min.
MS (ESI⁺): m/z = 490 [M+H]⁺
¹H-NMR (400MHz, DMSO-d₆): δ = 8.89 (bs, 2H), 8.12 (d, 1H), 7.55 (m, 3H), 7.29 (m, 2H), 7.20 (s, 1H), 6.88 (d, 1H), 6.68 (dd, 1H), 4.02 (bs, 2H), 3.77 (s, 3H), 3.71 (bs, 2H), 3.58 (bs, 4H).

### Beispiel 2

### N-(1-Methyl-2-{[4-(5-methylpyridin-2-yl)piperazin-1-yl]carbonyl}-1H-imidazol-4-yl)-N'-[4-(trifluormethoxy)phenyl]harnstoff

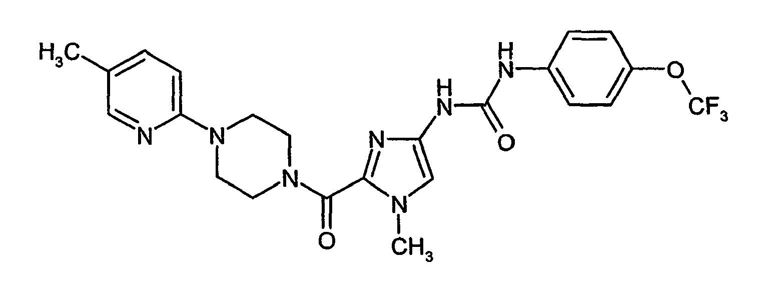

100 mg (0.29 mmol) Beispiel 13A werden in 2 mL DMF gelöst und mit 139 mg (0.44 mmol) O-(Benzotriazol-1-yl)-N,N,N',N'-tetramethyluroniumtetrafluoroborat (TBTU) und 53 mg (0.44 mmol) 4-Dimethylaminopyridin versetzt. Nach Zugabe von 103 mg (0.58 mmol) Beispiel 14A lässt man 4 h bei RT rühren. Das Reaktionsgemisch wird durch RP-HPLC gereinigt.
Ausbeute: 103 mg (70 % d. Th.). -
LC-MS (Methode 5): Rₜ = 2.01 min.
MS (ESI⁺): m/z = 504 [M+H]⁺
¹H-NMR (300MHz, DMSO-d₆): δ = 8.92 (bs, 2H), 7.99 (d, 1H), 7.54 (m, 2H), 7.42 (dd, 1H), 7.28 (m, 2H), 7.20 (s, 1H), 6.80 (d, 1H), 4.00 (bs, 2H), 3.77 (s, 3H), 3.72 (bs, 2H), 3.51 (bs, 4H), 2.16 (s, 3H).

### Beispiel 3

### N-(2-{[4-(5-Chlorpyridin-2-yl)piperazin-1-yl]carbonyl}-1-ethyl-1H-imidazol-4-yl)-N'-[4-(tnfluormethoxy)phenyl]harnstoff

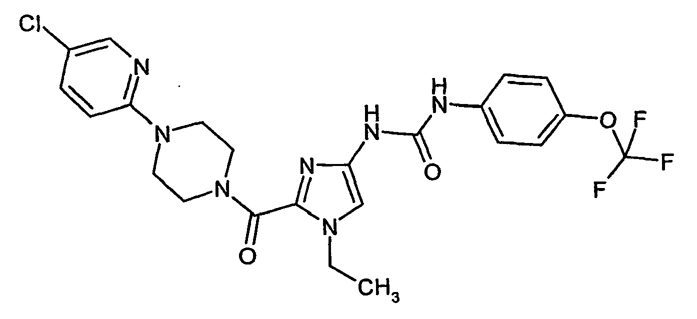

Die Herstellung erfolgt analog zu Beispiel 2.
Ausbeute: 55 mg (68 % d. Th.).
LC-MS (Methode 5): Rₜ = 2.76 min.
MS (ESI⁺): m/z= 538 [M+H]⁺
¹H-NMR (300MHz, DMSO-d₆): δ = 8.97 (bs, 1H), 8.92 (bs, 1H), 8.14 (d, 1H), 7.65 (dd, 1H), 7.54 (m, 2H), 7.28 (m, 2H), 7.24 (s, 1H), 6.92 (d, 1H), 4.16 (q, 2H), 3.97 (bs, 2H), 3.72 (bs, 2H), 3.59 (bs, 4H), 1.32 (t, 3H).

### Beispiel 4

### N-(2-{[4-(4-Methoxyphenyl)piperazin-1-yl]carbonyl}-1-methyl-1H-imidazol-4-yl)-N'-[4-(trifluormethoxy)phenyl]harnstoff

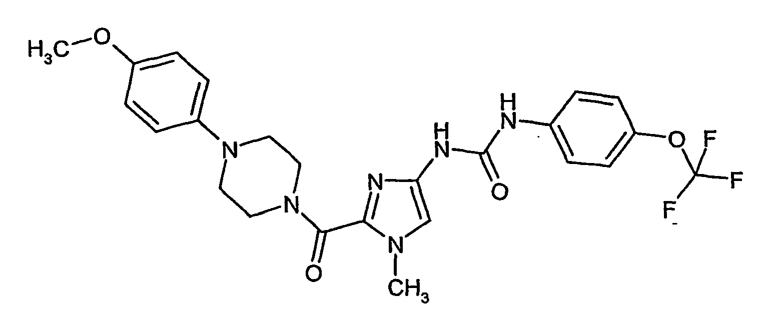

Die Herstellung erfolgt analog zu Beispiel 2.
Ausbeute: 35 mg (58 % d. Th.).
LC-MS (Methode 4): Rₜ = 2.24 min.
MS (ESI⁺): m/z = 519 [M+H]⁺
¹H-NMR (400MHz, DMSO-d₆): δ = 8.89 (bs, 2H), 7.53 (m, 2H), 7.28 (m, 2H), 7.19 (s, 1H), 6.92 (m, 2H), 6.84 (m, 2H), 4.05 (bs, 2H), 3.75 (m, 5H), 3.69 (s, 3H), 3.08 (bs, 4H).

### Beispiel 5

### N-[4-(Difluormethoxy)phenyl]-N'-(1-methyl-2-{[4-(5-methylpyridin-2-yl)piperazin-1-yl]-carbonyl}-1H-imidazol-4-yl)harnstoff

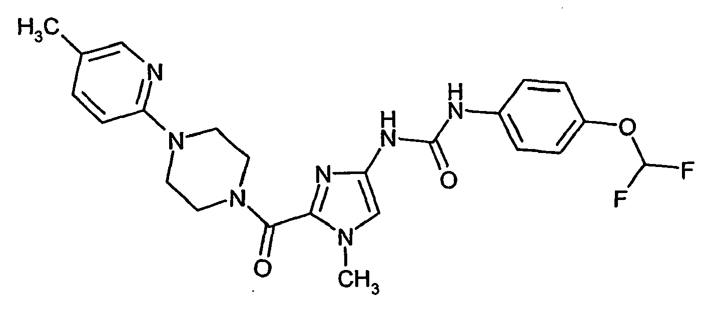

Die Herstellung erfolgt analog zu Beispiel 2 aus Beispiel 20A.
Ausbeute: 17 mg (29 % d. Th.).
LC-MS (Methode 5): Rₜ = 1.70 min.
MS (ESI⁺): m/z = 486 [M+H]⁺
¹H-NMR (400MHz, DMSO-d₆): δ = 8.84 (bs, 1H), 8.77 (bs, 1H), 7.98 (d, 1H), 7.47 (m, 2H), 7.42 (dd, 1H), 7.18 (s, 1H), 7.11 (t, 1H), 7.10 (m, 2H), 6.80 (d, 1H), 4.01 (bs, 2H), 3.77 (s, 3H), 3.71 (bs, 2H), 3.50 (bs, 4H), 2.16 (s, 3H).

Die Beispiele der Tabelle 1 werden analog zu Beispiel 2 hergestellt.

**Tabelle 1:**

| **Bsp.- Nr.** | **Struktur** | **Molmasse** | **MS (ESI) [M+H]⁺** | **LC-MS Rₜ [min] (Methode)** | **Ausgangsverbindung** | **Ausbeute (% d. Th.)** |
|---|---|---|---|---|---|---|
| **6** | 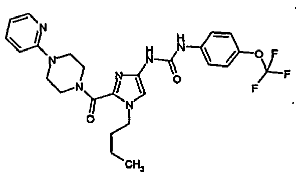 | 531.536 | 532 | 2.00 (4) | Beispiel 7A | 67 |
| **7** | 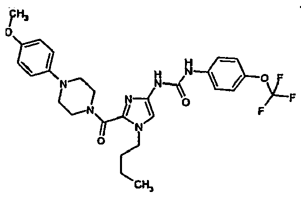 | 560.574 | 561 | 2.60 (4) | Beispiel 7A | 72 |
| **8** | 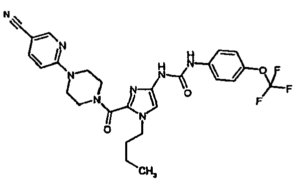 | 556.546 | 557 | 2.70 (2) | Beispiel 7A | 60 |
| **9** | 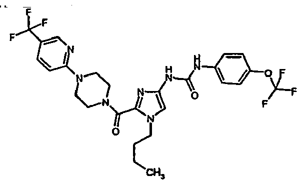 | 599.533 | 600 | 3.00 (5) | Beispiel 7A | 57 |
| **10** | 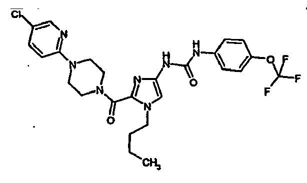 | 565.981 | 566 | 2.94 (5) | Beispiel 7A | 72 |
| **11** | 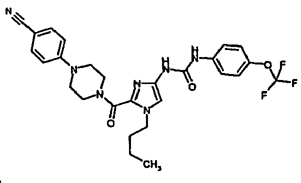 | 555.558 | 556 | 2.77 (2) | Beispiel 7A | 62 |
| **12** | 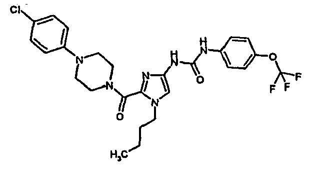 | 564.993 | 565 | 3.06 (5) | Beispiel 7A | 51 |
| **13** | 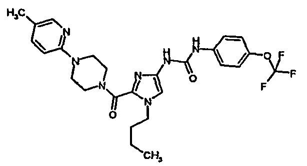 | 545.563 | 546 | 2.16 (5) | Beispiel 7A | 42 |
| **14** | 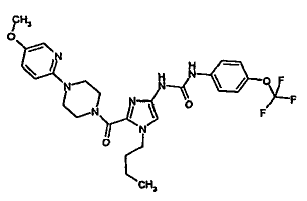 | 561.562 | 562 | 2.55 (5) | Beispiel 7A | 72 |
| **15** | 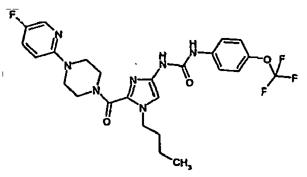 | 549.526 | 550 | 2.65 (4) | Beispiel 7A Beispiel 17A | 40 |
| **16** | 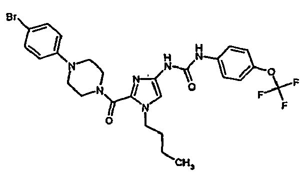 | 609.444 | 609 | 2.88 (4) | Beispiel 7A | 77 |
| **17** | 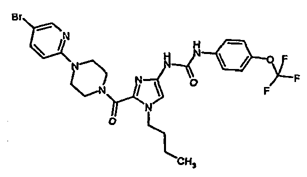 | 610.432 | 610 | 2.78 (4) | Beispiel 7A | 81 |
| **18** | 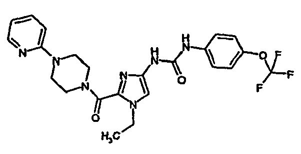 | 503.483 | 504 | 1.72 (4) | Beispiel 15A | 47 |
| **19** | 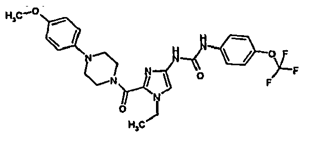 | 532.52 | 533 | 2.36 (4) | Beispiel 15A | 72 |
| **20** | 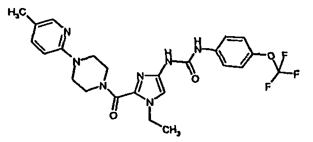 | 517.509 | 518 | 1.94 (5) | Beispiel 15A | 22 |
| **21** | 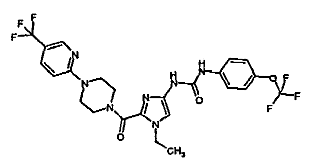 | 571.48 | 572 | 2.80 (5) | Beispiel 15A | 28 |
| **22** | 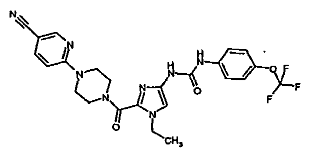 | 528.493 | 529 | 2.55 (5) | Beispiel 5A | 70 |
| **23** | 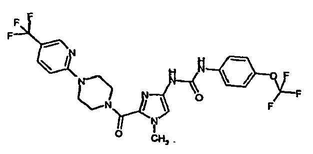 | 557.453 | 558 | 2.75 (5) | Beispiel 13A | 79 |
| **24** | 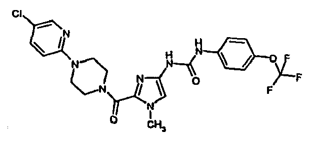 | 523.901 | 524 | 2.67 (5) | Beispiel 13A | 50 |
| **25** | 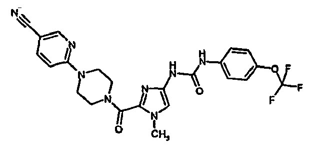 | 514.466 | 515 | 2.45 (5) | Beispiel 13A | 74 |
| **26** | 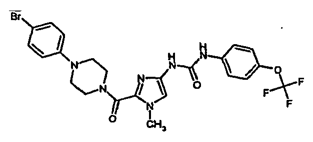 | 567.364 | 567 | 2.58 (4) | Beispiel 13A | 52 |
| **27** | 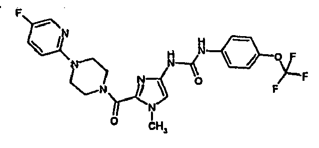 | 507.446 | 508 | 2.26 (4) | Beispiel 13A Beispiel 17A | 37 |
| **28** | 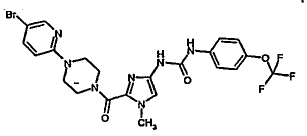 | 568.352 | 568 | 2.46 (4) | Beispiel 3A | 71 |
| **29** | 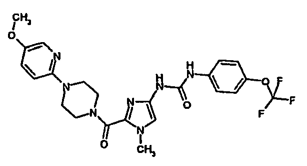 | 519.482 | 520 | 2.18 (5) | Beispiel 13A | 72 |
| **30** | 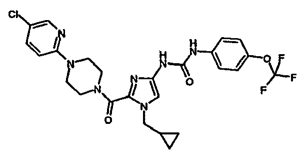 | 563.966 | 564 | 2.65 (4) | Beispiel 5A | 50 |
| **31** | 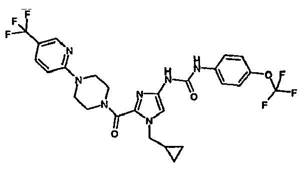 | 597.518 | 598 | 2.94 (5) | Beispiel 5A | 48 |
| **32** | 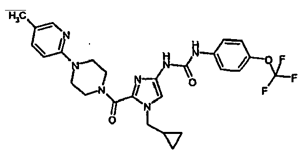 | 543.547 | 544 | 2.11 (5) | Beispiel 5A | 59 |
| **33** | 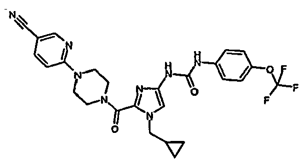 | 554.531 | 555 | 2.66 (5) | Beispiel 5A | 51 |
| **34** | 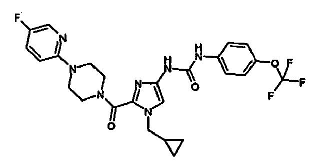 | 547.51 | 548 | 2.49 (4) | Beispiel 5A Beispiel 17A | 37 |
| **35** | 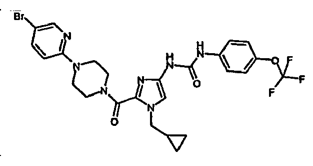 | 608.417 | 608 | 2.69 (4) | Beispiel 5A | 86 |
| **36** | 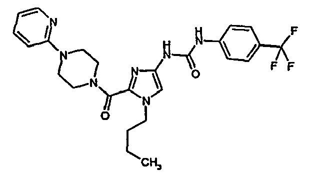 | 515.537 | 516 | 2.13 (2) | Beispiel 16A | 79 |
| **37** | 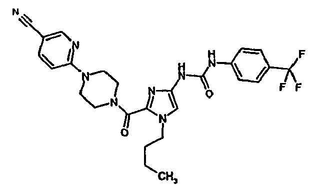 | 540.547 | 541 | 2.69 (2) | Beispiel 16A | 65 |
| **38** | 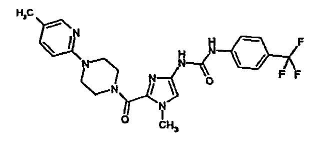 | 487.49 | 488 | 1.90 (5) | Beispiel 24A | 71 |
| **39** | 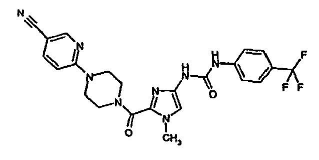 | 498.47 | 499 | 2.50 (5) | Beispiel 24A | 43 |

### Beispiel 40

### N-{1-(Cyclopropylmethyl)-2-[(4-pyridin-2-ylpiperazin-1-yl)carbonyl]-1H-imidazo1-4-yl}-N'-[4-(trifluormethoxy)phenyl]harnstoff

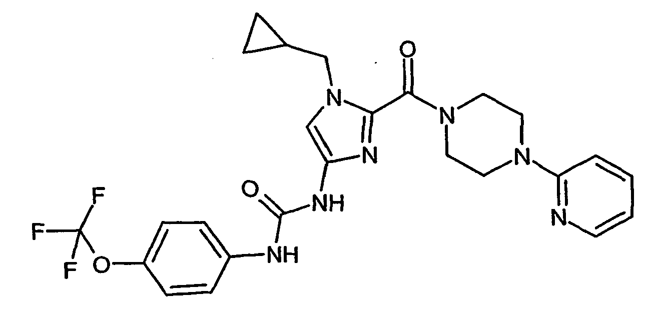

57.6 mg (0.15 mmol) Beispiel 5A werden in 0.5 mL DMF gelöst und mit 59.5 mg (0.15 mmol) *O-*(Benzotriazol-1-yl)-*N*,*N*,*N*',*N*'-tetramethyluronium-Hexafluorphosphat (HBTU) und 15 mg (0.15 mmol) Triethylamin versetzt. Nach Zugabe von 49 mg (0.3 mmol) N-(2-Pyridyl)piperazin lässt man 16 h bei RT rühren. Das Reaktionsgemisch wird durch RP-HPLC gereinigt.
Ausbeute: 46 mg (58 % d. Th.).
LC-MS (Methode 5): Rₜ = 2.08 min.
MS (ESI⁺): m/z = 530 [M+H]⁺ -
¹H-NMR (300MHz, DMSO-d₆): δ = 8.9 (s, 2H), 8.15 (d, 1H), 7.6-7.5 (m, 3H), 7.25 (m, 3H), 6.85 (d, 1H), 6.7 (dd, 1H), 4.05 (d, 2H), 4.00 (bs, 2H), 3.75 (bs, 2H), 3.6-3.5 (m, 4H), 1.75 (m, 1H), 0.5 (q, 2H), 0.3 5 (q, 2H).

Die Beispiele der Tabelle 2 werden analog zu Beispiel 40 hergestellt.

**Tabelle 2:**

| **Bsp.- Nr.** | **Struktur** | **Molmasse** | **MS (EI) [M+H]⁺** | **LC-MS Rₜ [min] (Methode)** | **Ausgangsverbindung** | **Ausbeute [mg] (% d. Th.)** |
|---|---|---|---|---|---|---|
| **41** | 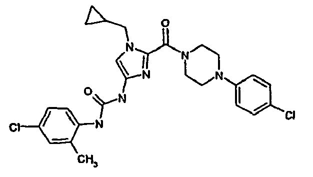 | 527.458 | 527 | 2.9 (2) | Beispiel 4A | 43.6 (49) |
| **42** | 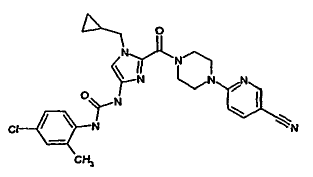 | 519.01 | 519 | 2.55 (2) | Beispiel 4A | 23.3 (30) |
| **43** | 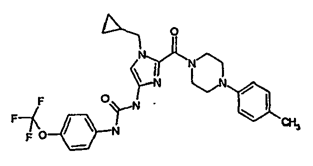 | 542.566 | 543 | 2.88 (2) | Beispiel 5A | 21.6 (24) |
| **44** | 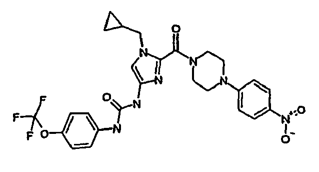 | 573.536 | 574 | 2.75 (2) | Beispiel 5A | 24.4 (26) |
| **45** | 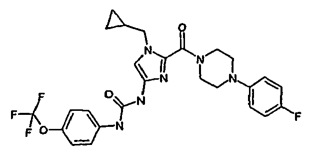 | 546.529 | 547 | 2.8 (2) | Beispiel 5A | 28.5 (33) |
| **46** | 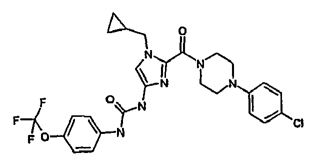 | 562.984 | 563 | 2.95 (2) | Beispiel 5A | 42.4 (47) |
| **47** | 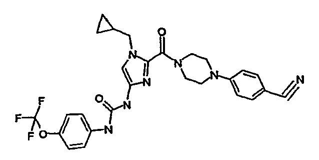 | 553.549 | 554 | 2.7 (2) | Beispiel 5A | 9.2 (9) |
| **48** | 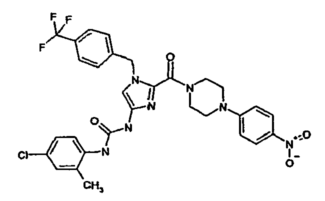 | 642.042 | 642 | 2.93 (2) | Beispiel 9A | 25.2 (21) |
| **49** | 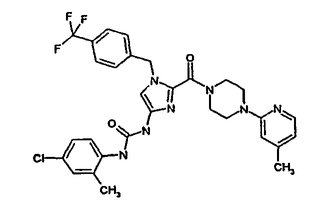 | 612.06 | 612 | 2.3 (2) | Beispiel 9A | 2.1 (2) |
| **50** | 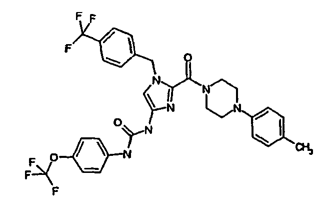 | 646.598 | 647 | 3.09 (2) | Beispiel 8A | 36.4 (38) |
| **51** | 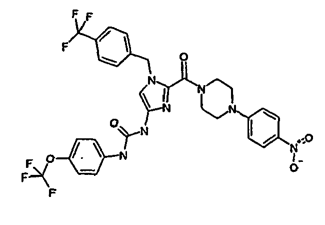 | 677.568 | 678 | 2.97 (2) | Beispiel 8A | 16.3 (15) |

### Beispiel 52

### N-(3,5-Difluorphenyl)-N'-{1-ethyl-2-[(4-pyridin-2-ylpiperazin-1-yl)carbonyl]-1H-imidazol-4-yl}harnstoff

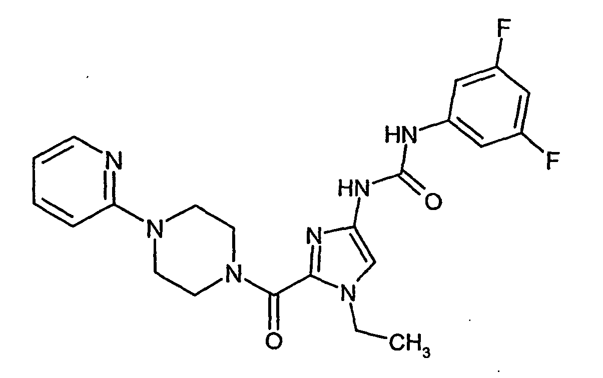

Eine Lösung von 50 mg (0.15 mmol) 1-[(1-Ethyl-4-nitro-1H-imidazol-2-yl)carbonyl]-4-(pyridin-2-yl)piperazin in 6 mL absolutem THF wird zunächst mit einer Spatelspitze Raney-Nickel und anschließend mit 11 mg (0.23 mmol) Hydrazinhydrat versetzt und anschließend 1h nachgerührt. Zu der Rohlösung wird Natriumsulfat gegeben und anschließend über Kieselgur filtriert und mit Methylenchlorid nachgewaschen. Das Filtrat wird im Vakuum eingeengt und in 6 mL THF wieder aufgenommen und mit 28 mg (0.18 mmol) Difluorphenylisocyanat und 2 mg 1,4-Diazabicyclo(2.2.2)octan versetzt und bei Raumtemperatur gerührt. Nach 1h wird das Lösemittel abrotiert und der Rückstand mittels präparativer HPLC gereinigt. Man erhält 20 mg (29 % d.Th.) des Produktes.
HPLC (Methode 10): Rₜ = 3.94 min.
MS (ESI⁺): m/z = 456 [M+H]⁺
¹H-NMR (300MHz, DMSO-d₆): δ = 8.5 (2s, 2H), 8.1 (m, 1H), 7.55 (m, 1H), 7.25 (s, 1H), /.15 (m, 2H), 6.9-6.65 (m, 3H), 4.2 (q, 2H), 3.9 (m, 2H), 3.7 (m, 2H), 3.55 (m, 4H), 1.3 (t, 3H).

Die Beispiele der Tabelle 3 werden analog zu Beispiel 52 hergestellt, mit Ausnahme von Beispiel 57, das analog zu Beispiel 2 hergestellt wird.

**Tabelle 3:**

| **Bsp.- Nr.** | **Struktur** | **Molmasse** | **MS (ESI) [M+H]⁺** | **HPLC Rₜ [min] (Methode 10)** | **Ausgangsverbindung** | **Ausbeute [mg] (% d. Th.)** |
|---|---|---|---|---|---|---|
| **53** | 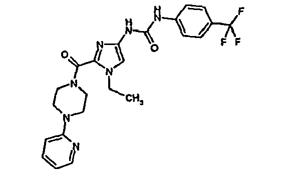 | 487.48 | 488 | 4.118 | Beispiel 21A | 40 (51) |
| **54** | 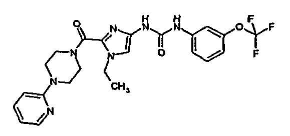 | 503.48 | 504 | 4.175 | Beispiel 21A | 45 (57) |
| **55** | 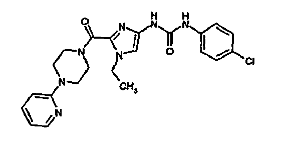 | 453.93 | 454 | 3.937 | Beispiel 21A | 43 (63) |
| **56** | 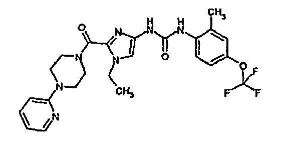 | 517.51 | 518 | 4.182 | Beispiel 21A | 35 (45) |
| **57** | 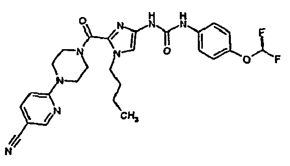 | 538.56 | 539 | 4.588 | Beispiel 22A | 40 (82) |
| **58** | 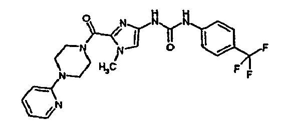 | 473.46 | 474 | 4.014 | Beispiel 23A | 40 (53) |
| **59** | 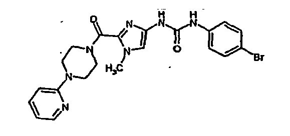 | 484.36 | 484 | 3.899 | Beispiel 23A | 51 (66) |
| **60** | 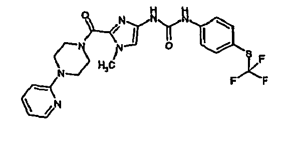 | 505.52 | 506 | 4.260 | Beispiel 23A | 57 (70) |

### B. Bewertung der physiologischen Wirksamkeit

Die *in vitro*-Wirkung der erfindungsgemäßen Verbindungen kann in folgenden Assays gezeigt werden:

### Anti-HCMV- (Anti-Humanes Cytomegalie-Virus) Zytopathogenitätstests

Die Testverbindungen werden als 50 millimolare (mM) Lösungen in Dimethysulfoxid (DMSO) eingesetzt. Ganciclovir, Foscarnet und Cidofovir dienen als Referenzverbindungen. Nach der Zugabe von jeweils 2 µl der 50, 5, 0.5 und 0.05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x 10⁴ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0.001 - 0.002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0.0005 µM. Die Platten werden 6 Tage bei 37°C /5% CO₂ inkubiert, d.h. bis in den Viruskontrollen alle Zellen infiziert sind (100 % cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque multiplier der Firma Technomara) visuell ausgewertet.

Die folgenden Daten können von den Testplatten ermittelt werden:
CC₅₀ (NHDF) = maximale Substanzkonzentration in µM, bei der im Vergleich zur unbehandelten Zellkontrolle keine sichtbaren cytostatischen Effekte auf die Zellen erkennbar sind;
EC₅₀ (HCMV) = Substanzkonzentration in µM, die den CPE (cytopathischen Effekt) um 50 % im Vergleich zur unbehandelten Viruskontrolle hemmt;
SI (Selektivitätsindex) = CC₅₀ (NHDF) / EC₅₀ (HCMV).

Repräsentative in-vitro-Wirkdaten für die erfindungsgemäßen Verbindungen sind in Tabelle A wiedergegeben:

**Tabelle A**

| **Beispiel-Nr.** | **NHDF** **CC₅₀ [nM]** | **HCMV** **EC₅₀ [nM]** | **SI HCMV** |
|---|---|---|---|
| **1** | 43.5 | 3.0 | 14500 |
| **2** | 10.9 | 0.75 | 14530 |
| **25** | 12.5 | 4.8 | 2600 |
| **29** | 34.0 | 0.95 | 35790 |
| **32** | 5.3 | 0.85 | 6240 |

Die Eignung der erfindungsgemäßen Verbindungen zur Behandlung von HCMV-Infektionen kann im folgenden Tiermodell gezeigt werden:

### HCMV Xenoeraft-Gelfoam^{®}-Modell

### Tiere:

3-4 Wochen alte weibliche immundefiziente Mäuse (16-18 g), Fox Chase SCID oder Fox Chase SCID-NOD oder SCID-beige werden von kommerziellen Züchtern (Bomholtgaard, Jackson) bezogen. Die Tiere werden unter sterilen Bedingungen (einschließlich Streu und Futter) in Isolatoren gehalten.

### Virusanzucht:

Humanes Cytomegalievirus (HCMV), Stamm Davis, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0.01 werden die virusinfizierten Zellen 5-7 Tage später geerntet und in Gegenwart von Minimal Essential Medium (MEM), 10 % foetalem Kälberserum (FKS) mit 10 % DMSO bei -40°C aufbewahrt. Nach serieller Verdünnung der virusinfizierten Zellen in Zehnerschritten erfolgt die Titerbestimmung auf 24-Well-Platten konfluenter NHDF-Zellen nach Vitalfärbung mit Neutralrot oder Fixierung und Färbung mit einem Formalin-Giemsa Gemisch (wie oben beschrieben).

### Vorbereitung der Schwämme, Transplantation, Behandlung und Auswertung:

1x1x1 cm große Kollagenschwämme (Gelfoam^{®}; Fa. Peasel & Lorey, Best.-Nr. 407534; K.T. Chong et al., Abstracts of 39th Interscience Conference on Antimicrobial Agents and Chemotherapy, 1999, S. 439; P.M. Kraemer et al., Cancer Research 1983, (43): 4822-4827) werden zunächst mit Phosphat-gepufferter Saline (PBS) benetzt, die eingeschlossenen Luftblasen durch Entgasen entfernt und dann in MEM + 10 % FKS aufbewahrt. 1 x 10⁶ virusinfizierte NHDF-Zellen (Infektion mit HCMV-Davis M.O.I = 0.01) werden 3 Stunden nach Infektion abgelöst und in 20 µl MEM, 10 % FKS auf einen feuchten Schwamm getropft. Optional werden nach 12-13 Stunden auf die infizierten Schwämme 5 ng/µl basic Fibroblast Growth Factor (bFGF) in 25 µl PBS / 0.1% BSA / 1 mM DTT aufgebracht und 1 Stunde inkubiert. Zur Transplantation werden die immundefizienten Mäuse mit Avertin oder einem Gemisch aus Azepromazin-Xylazin und Ketamin narkotisiert, das Rückenfell mit Hilfe eines Trockenrasierers entfernt, die Oberhaut 1-2 cm geöffnet, entlastet und die feuchten Schwämme unter die Rückenhaut transplantiert. Die Operationswunde wird mit Gewebekleber verschlossen. 24 Stunden nach der Transplantation werden die Mäuse über einen Zeitraum von 8 Tagen dreimal täglich (7.00 Uhr und 14.00 Uhr und 19.00 Uhr), zweimal täglich (8.00 Uhr und 17.00 Uhr), oder einmal täglich (14.00 Uhr) peroral mit Substanz behandelt. Die Dosis beträgt 3 oder 10 oder 30 oder 100 mg/kg Körpergewicht, das Applikationsvolumen 10 mL/kg Körpergewicht. Die Formulierung der Substanzen erfolgt in Form einer 0,5 %igen Tylosesuspension optional mit 2 % DMSO. 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U /1.5 mL) aus dem Schwamm freigesetzt und in Gegenwart von MEM, 10 % foetalem Kälberserum, 10 % DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot oder nach Fixierung und Färbung mit einem Formalin-Giemsa Gemisch (wie oben beschrieben). Ermittelt wird die Anzahl infektiöser Viruspartikel nach Substanzbehandlung im Vergleich zur placebobehandelten Kontrollgruppe.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5 %-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 mL orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6 h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die erfindungsgemäße Verbindung wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefühlt. Diese werden mit Infusionsstopfen und Bördelkappen verschlossen.

## Patentansprüche

1. Verbindung der Formel 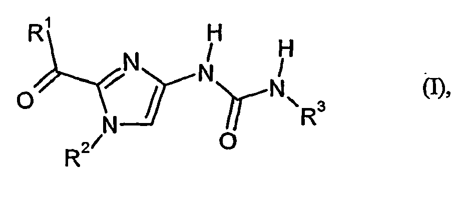 in welcher
R¹ für eine Gruppe der Formel 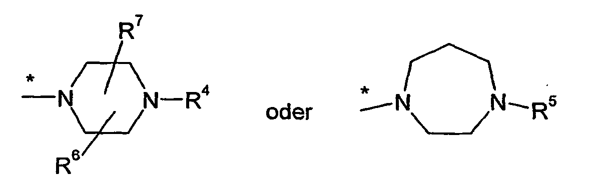 steht,
wobei
* für die Anknüpfstelle an die Carbonyl-Gruppe steht,
R⁴ für Phenyl oder 5- oder 6-gliedriges Heteroaryl steht,
worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
R⁵ für Phenyl oder 5- oder 6-gliedriges Heteroaryl steht,
worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, C₁-C₆-Akyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
und
R⁶ und R⁷ unabhängig voneinander für Wasserstoff, Methyl oder Ethyl stehen,
R² für C₁-C₆-Alkyl steht,
wobei Alkyl substituiert sein kann mit einem Substituenten, wobei der Substituent ausgewählt wird aus der Gruppe, bestehend aus C₃-C₆-Cycloalkyl, C₆-C₁₀-Aryl und 5- oder 6-gliedriges Heteroaryl,
worin Cycloalkyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluor-methoxy, Trifluormethylthio, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
R³ für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, Monofluormethoxy, Trifluormethylthio, C₁-C₆-Alkyl und C₁-C₆-Alkoxy,
oder eines ihrer Salze, ihrer Solvate oder der Solvate ihrer Salze.

2. Verbindung nach Anspruch 1, **dadurch gekennzeichnet, dass**
R¹ für eine Gruppe der Formel 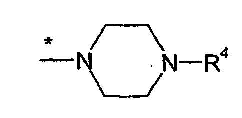 steht,
wobei
* für die Anknüpfstelle an die Carbonyl-Gruppe steht,
R⁴ für Phenyl oder 5- oder 6-gliedriges Heteroaryl steht,
worin Phenyl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
R² für C₁-C₆-Alkyl steht,
wobei Alkyl substituiert sein kann mit einem Substituenten, wobei der Substituent ausgewählt wird aus der Gruppe, bestehend aus C₃-C₆-Cycloalkyl und Phenyl,
worin Cycloalkyl und Phenyl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, Monofluormethoxy, Trifluormethylthio, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
R³ für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, Monofluormethoxy, Trifluormethylthio, C₁-C₆-Alkyl und C₁-C₆-Alkoxy.

3. Verbindung nach Anspruch 1 oder 2, **dadurch gekennzeichnet, dass**
R¹ für eine Gruppe der Formel 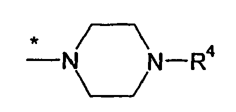 steht,
wobei
* für die Anknüpfstelle an die Carbonyl-Gruppe steht,
R⁴ für Phenyl oder Pyridyl steht,
worin Phenyl und Pyridyl substituiert sein können mit 1 bis 3 Substituenten, wobei die Substituenten unabhängig voneinander ausgewählt werden aus der Gruppe, bestehend aus Halogen, Nitro, Cyano, Trifluormethyl, Difluormethyl, Trifluormethoxy, Difluormethoxy, Monofluormethoxy, C₁-C₄-Alkyl und C₁-C₄-Alkoxy,
R² für Methyl, Ethyl oder n-Butyl steht,
wobei Methyl, Ethyl und n-Butyl substituiert sein können mit einem Substituenten, wobei der Substituent ausgewählt wird aus der Gruppe, bestehend aus Cyclopropyl und Phenyl,
worin Phenyl substituiert sein kann mit einem Substituenten Trifluormethyl,
R³ für Phenyl steht,
wobei Phenyl substituiert sein kann mit 1 bis 3 Substituenten, wobei die Substituenten unabhängig voneinander ausgewählt werden aus der Gruppe, bestehend aus Fluor, Chlor, Trifluormethoxy, Difluormethoxy, Trifluormethylthio und Methyl.

4. Verfahren zur Herstellung einer Verbindung der Formel (I) nach Anspruch 1, **dadurch gekennzeichnet, dass**
nach Verfahren [A] eine Verbindung der Formel 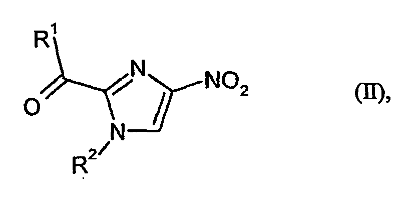 in welcher
R¹ und R² die in Anspruch 1 angegebene Bedeutung haben,
in der ersten Stufe mit einem Reduktionsmittel und in der zweiten Stufe in Gegenwart eines Kohlensäurederivates mit einer Verbindung der Formel
H₂N-R³ (III),
in welcher
R³ die in Anspruch 1 angegebene Bedeutung hat,
oder
nach Verfahren [B] eine Verbindung der Formel (II) in der ersten Stufe mit einem Reduktionsmittel und in der zweiten Stufe mit einer Verbindung der Formel
OCN-R³ (IV),
in welcher
R³ die in Anspruch 1 angegebene Bedeutung hat,
oder
nach Verfahren [C] eine Verbindung der Formel 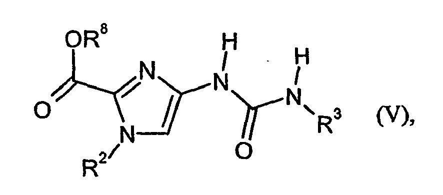 in welcher
R² und R³ die in Anspruch 1 angegebene Bedeutung haben, und
R⁸ für Methyl oder Ethyl steht,
in der ersten Stufe mit einer Base und in der zweiten Stufe mit einer Verbindung der Formel
R¹-H (VI),
in welcher
R¹ die in Anspruch 1 angegeben Bedeutung hat,
in Gegenwart von Dehydratisierungsreagenzien umgesetzt wird.

5. Verbindung nach einem der Ansprüche 1 bis 3 zur Behandlung und/oder Prophylaxe von Krankheiten.

6. Arzneimittel enthaltend eine Verbindung nach einem der Ansprüche 1 bis 3 in Kombination mit mindestens einem inerten, nichttoxischen, pharmazeutisch geeigneten Hilfsstoff.

7. Verwendung einer Verbindung nach einem der Ansprüche 1 bis 3 zur Herstellung eines Arzneimittels zur Behandlung und/oder Prophylaxe von Virusinfektionen.

8. Verwendung nach Anspruch 7, **dadurch gekennzeichnet, dass** die Virusinfektion eine Infektion mit dem humanen Cytomegalievirus (HCMV) oder einem anderen Vertreter der Gruppe der Herpes viridae ist.

9. Arzneimittel nach Anspruch 6 zur Behandlung und/oder Prophylaxe von Virusinfektionen.

10. Verwendung einer antiviral wirksamen Menge mindestens einer Verbindung nach einem der Ansprüche 1 bis 3, eines Arzneimittels nach Anspruch 6 oder eines nach Anspruch 7 oder 8 erhaltenen Arzneimittels zur Herstellung eines Artneimittels zur Bekämpfung von Virusinfektionen in Menschen und Tieren.

## Claims

1. Compound of formula 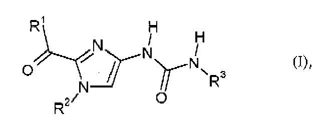 in which
R¹ represents a group of formula 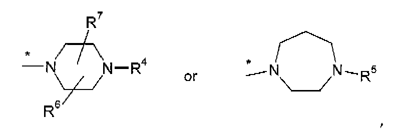 whereby
* represents the linkage site to the carbonyl group,
R⁴ represents phenyl or 5- or 6-membered heteroaryl,
wherein phenyl and heteroaryl may be substituted with 1 to 3 substituents, whereby the substituents are selected independently of one another from the group consisting of halogen, hydroxy, oxo, nitro, cyano, trifluoromethyl, difluoromethyl, trifluoromethoxy, difluoromethoxy, monofluoromethoxy, trifluoromethylthio, C₁-C₆-alkyl, C₁-C₆-alkoxy, hydroxycarbonyl, C₁-C₆-alkoxycarbonyl, amino, C₁-C₆-alkylamino, aminocarbonyl and C₁-C₆-alkylaminocarbonyl,
R⁵ represents phenyl or 5- or 6-membered heteroaryl,
wherein phenyl and heteroaryl may be substituted with 1 to 3 substituents, whereby the substituents are selected independently of one another from the group consisting of halogen, hydroxy, oxo, nitro, cyano, trifluoromethyl, difluoromethyl, trifluoromethoxy, difluoromethoxy, monofluoromethoxy, trifluoromethylthio, C₃-C₆-alkyl, C₁-C₆-alkoxy, hydroxycarbonyl, C₁-C₆-alkoxycarbonyl, amino, C₁-C₆-alkylamino, aminocarbonyl and C₁-C₆-alkylaminocarbonyl,
and
R⁶ and R⁷ independently of one another represent hydrogen, methyl or ethyl,
R² represents C₁-C₆-alkyl,
whereby alkyl may be substituted with a substituent, whereby the substituent is selected from the group consisting of C₃-C₆-cycloalkyl, C₆-C₁₀-aryl and 5- or 6-membered heteroaryl,
wherein cycloalkyl, aryl and heteroaryl may be substituted with 1 to 3 substituents, whereby the substituents are selected independently of one another from the group consisting of halogen, hydroxy, oxo, nitro, cyano, trifluoromethyl, difluoromethyl, trifluoromethoxy, difluoromethoxy, monofluoromethoxy, trifluoromethylthio, C₁-C₆-alkyl, C₁-C₆-alkoxy, hydroxycarbonyl, C₁-C₆-alkoxycarbonyl, amino, C₁-C₆-alkylamino, aminocarbonyl and C₁-C₆-alkylaminocarbonyl,
R³ represents phenyl,
whereby phenyl may be substituted with 1 to 3 substituents, whereby the substituents are selected independently of one another from the group consisting of halogen, hydroxy, trifluoromethyl, difluoromethyl, trifluoromethoxy, difluoromethoxy, monofluoromethoxy, trifluoromethylthio, C₁-C₆-alkyl and C₁-C₆-alkoxy,
or one of its salts, its solvates or the solvates of its salts.

2. Compound according to Claim 1, **characterized in that**
R¹ represents a group of formula 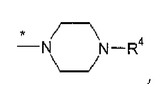 whereby
* represents the linkage site to the carbonyl group,
R⁴ represents phenyl or 5- or 6-membered heteroaryl,
wherein phenyl and heteroaryl may be substituted with 1 to 3 substituents, whereby the substituents are selected independently of one another from the group consisting of halogen, hydroxy, oxo, nitro, cyano, trifluoromethyl, difluoromethyl, trifluoromethoxy, difluoromethoxy, monofluoromethoxy, trifluoromethylthio, C₁-C₆-alkyl, C₁-C₆-alkoxy, hydroxycarbonyl, C₁-C₆-alkoxycarbonyl, amino, C₁-C₆-alkylamino, aminocarbonyl and C₁-C₆-alkylaminocarbonyl,
R² represents C₁-C₂-alkyl,
whereby alkyl may be substituted with a substituents, whereby the substituent is selected from the group consisting of C₃-C₆-cycloalkyl and phenyl,
wherein cycloalkyl and phenyl may be substituted with 1 to 3 substituents, whereby the substituents are selected independently of one another from the group consisting of halogen, hydroxy, oxo, nitro, cyano, trifluoromethyl, difluoromethyl, trifluoromethoxy, difluoromethoxy, monofluoromethoxy, trifluoromethylthio, C₁-C₆-alkyl, C₁-C₆-alkoxy, hydroxycarbonyl, C₁-C₆-alkoxycarbonyl, amino, C₁-C₆-alkylamino, aminocarbonyl and C₁-C₆-alkylaminocarbonyl,
R³ represents phenyl,
whereby phenyl may be substituted with 1 to 3 substituents, whereby the substituents are selected independently of one another from the group consisting of halogen, hydroxy, trifluoromethyl, difluoromethyl, trifluoromethoxy, difluoromethoxy, monofluoromethoxy, trifluoromethylthio, C₁-C₆-alkyl and C₁-C₆-alkoxy.

3. Compound according to Claim 1 or 2, **characterized in that**
R¹ represents a group of formula 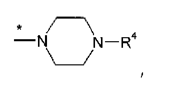 whereby
* represents the linkage site to the carbonyl group,
R⁴ represents phenyl or pyridyl,
wherein phenyl and pyridyl may be substituted with 1 to 3 substituents, whereby the substituents are selected independently of one another from the group consisting of halogen, nitro, cyano, trifluoromethyl, difluoromethyl, trifluoromethoxy, difluoromethoxy, monofluoromethoxy, C₁-C₄-alkyl and C₁-C₄-alkoxy,
R² represents methyl, ethyl or n-butyl,
whereby methyl, ethyl and n-butyl may be substituted with a substituent, whereby the substituent is selected from the group consisting of cyclopropyl and phenyl,
wherein phenyl may be substituted with a trifluoromethyl substituent,
R³ represents phenyl,
whereby phenyl may be substituted with 1 to 3 substituents, whereby the substituents are selected independently of one another from the group consisting of fluorine, chlorine, trifluoromethoxy, difluoromethoxy, trifluoromethylthio and methyl.

4. Method for preparing a compound of formula (I) according to Claim 1,
**characterized in that** according to method [A] a compound of formula 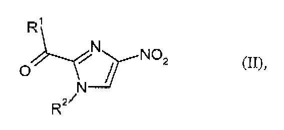 in which
R¹ and R² have the meaning indicated in Claim 1,
is reacted in the first step with a reducing agent and in the second step in the presence of a carbonic acid derivative with a compound of formula
H₂N-R³ (III),
in which
R³ has the meaning indicated in Claim 1,
or
according to method [R] a compound of formula (II) is reacted in the first step with a reducing agent and in the second step with a compound of formula
OCN-R³ (IV),
in which
R³ has the meaning indicated in Claim 1,
or
according to method [C] a compound of formula 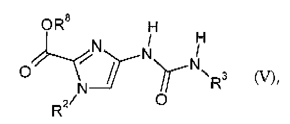 in which
R² and R³ have the meaning indicated in Claim 1, and
R⁸ represents methyl or ethyl,
is reacted in the first step with a base and in the second step with a compound of formula
R¹-H (VI),
in which
R¹ has the meaning indicated in Claim 1,
in the presence of dehydrating reagents.

5. Compound according to any one of Claims 1 to 3 for the treatment and/or prophylaxis of diseases.

6. Medicament, comprising a compound according to any one of Claims 1 to 3 in combination with at least one inert nontoxic, pharmaceutically acceptable excipient.

7. Use of a compound according to any one of Claims 1 to 3 for the production of a medicament for the treatment and/or prophylaxis of viral infections.

8. Use according to Claim 7, **characterized in that** the viral infection is an infection with the human cytomegalovirus (HCMV) or another representative of the group of Herpes viridae.

9. Medicament according to Claim 6 for the treatment and/or prophylaxis of viral infections.

10. Use of an antivirally effective amount of at least one compound according to any one of Claims 1 to 3, a medicament according to Claim 6 or a medicament obtained according to Claim 7 or 8 for the production of a medicament for controlling viral infections in humans and animals.

## Revendications

1. Composé de formule 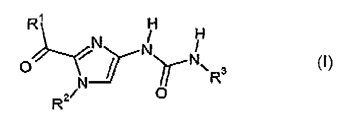 dans laquelle
R¹ représente un groupe de formule 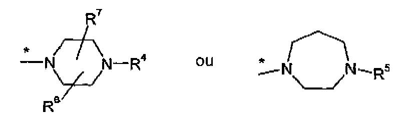
où
* représente le point de rattachement au groupe carbonyle,
R⁴ représente le phényle ou un hétéroaryle à 5 ou 6 atomes,
le phényle et l'hétéroaryle pouvant être substitués par 1 à 3 substituants choisis indépendamment les uns des autres dans le groupe constitué par les halogènes, hydroxy, oxo, nitro, cyano, trifluorométhyle, difluorométhyle, trifluorométhoxy, difluorométhoxy, monofluorométhoxy, trifluorométhylthio, alkyle en C₁ à C₆, alcoxy en C₁ à C₆, hydroxycarbonyle, alcoxycarbonyle en C₁ à C₆, amino, alkylamino en C₁ à C₆, aminocarbonyle et alkylaminocarbonyle en C₁ à C₈,
R⁵ représente le phényle ou un hétéroaryle à 5 ou 6 atomes,
le phényle et l'hétéroaryle pouvant être substitués par 1 à 3 substituants choisis indépendamment les uns des autres dans le groupe constitué par les halogènes, hydroxy, oxo, nitro, cyano, trifluorométhyle, difluorométhyle, trifluorométhoxy, difluorométhoxy, monofluorométhoxy, trifluorométhylthio, alkyle en C₁ à C₆, alcoxy en C₁ à C₆, hydroxycarbonyle, alcoxycarbonyle en C₁ à C₆ amino, alkylamino en C₁ à C₆, aminocarbonyle et alkylaminocarbonyle en C₁ à C₆,
et
R⁶ et R⁷ représentent indépendamment l'un de l'autre l'hydrogène, le méthyle ou l'éthyle,
R² représente un alkyle en C₁ à C₆,
l'alkyle pouvant être substitué par un substituant choisi dans le groupe constitué par cycloalkyle en C₃ à C₆, aryle en C₆ à C₁₀ et hétéroaryle à 5 ou 6 atomes,
le cycloalkyle, l'aryle et l'hétéroaryle pouvant être substitués par 1 à 3 substituants choisis indépendamment les uns des autres dans le groupe constitué par les halogènes, hydroxy, oxo, nitro, cyano, trifluorométhyle, difluorométhyle, trifluorométhoxy, difluorométhoxy, monofluorométhoxy, trifluorométhylthio, alkyle en C₁ à C₆, alcoxy en C₁ à C₆, hydroxycarbonyle, alcoxycarbonyle en C₁ à C₆, amino, alkylamino en C₁ à C₆, aminocarbonyle et alkylaminocarbonyle en C₁ à C₆
R³ représente le phényle,
le phényle pouvant être substitué par 1 à 3 substituants choisis indépendamment les uns des autres dans le groupe constitué par les halogènes, hydroxy, trifluorométhyle, difluorométhyle, trifluorométhoxy, difluorométhoxy, monofluorométhoxy, trifluorométhylthio, alkyle en C₁ à C₆ et alcoxy en C₁ à C₆,
ou un de ses sels, de ses solvates ou des solvates de ses sels.

2. Composé selon la revendication 1, **caractérisé en ce que**
R¹ représente un groupe de formule 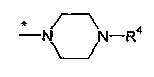 où
* représente le point de rattachement au groupe carbonyle,
R⁴ représente le phényle ou un hétéroaryle à 5 ou 6 atomes,
le phényle et l'hétéroaryle pouvant être substitués par 1 à 3 substituants choisis indépendamment les uns des autres dans le groupe constitué par les halogènes, hydroxy, oxo, nitro, cyano, trifluorométhyle, difluorométhyle, trifluorométhoxy, difluorométhoxy, monofluorométhoxy, trifluorométhylthio, alkyle en C₁à C₆, alcoxy en C₁ à C₆ hydroxycarbonyle, alcoxycarbonyle en C₁ à C₆, amino, alkylamino en C₁à C₆ aminocarbonyle et alkylaminocarbonyle en C₁ à C₆
R² représente un alkyle en C₁ à C₆,
l'alkyle pouvant être substitué par un substituant choisi dans le groupe constitué par cycloalkyle en C₃ à C₆ et phényle,
le cycloalkyle et le phényle pouvant être substitués par 1 à 3 substituants choisis indépendamment les uns des autres dans le groupe constitué par les halogènes, hydroxy, oxo, nitro, cyano, trifiluorométhyle, difluorométhyle, trifluorométhoxy, difluorométhoxy, monofluorométhoxy, trifluorométhylthio, alkyle en C₁à C₆, alcoxy en C₁ à C₆ hydroxycarbonyle, alcoxycarbonyle en C₁ à C₆, amino, alkylamino en C₁ à C₆ aminocarbonyle et alkylaminocarbonyle en C₁ à C₆,
R³ représente le phényle,
le phényle pouvant être substitué par 1 à 3 substituants choisis indépendamment les uns des autres dans le groupe constitué par les halogènes, hydroxy, trifluorométhyle, difluorométhyle, trifluorométhoxy, difluorométhoxy, monofluorométhoxy, trifluorométhylthio, alkyle en C₁ à C₆ et alcoxy en C₁ à C₆

3. Composé selon la revendication 1 ou la revendication 2, **caractérisé en ce que**
R¹ représente un groupe de formule 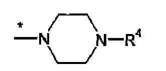
où
* représente le point de rattachement au groupe carbonyle,
R⁴ représente le phényle ou le pyridyle,
le phényle et le pyridyle pouvant être substitués par 1 à 3 substituants choisis indépendamment les uns des autres dans le groupe constitué par les halogènes, nitro, cyano, trifluorométhyle, difluorométhyle, trifluorométhoxy, difluorométhoxy, monofluorométhoxy, alkyle en C₁ à C₄ et alcoxy en C₁ à C₄,
R² représente le méthyle, l'éthyle ou le n-butyle,
le méthyle, l'éthyle et le n-butyle pouvant être substitués par un substituant choisi dans le groupe constitué par cyclopropyle et phényle,
le phényle pouvant être substitué par un substituant trifluorométhyle,
R³ représente le phényle,
le phényle pouvant être substitué par 1 à 3 substituants choisis indépendamment les uns des autres dans le groupe constitué par fluoro, chloro, trifluorométhoxy, difluorométhoxy, trifiluorométhylthio et méthyle.

4. Procédé de préparation d'un composé de formule (I) selon la revendication 1, **caractérisé en ce que**
suivant le procédé [A], on fait réagir un composé de formule 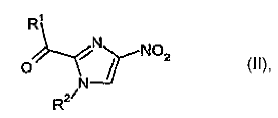 dans laquelle
R¹ et R² ont la signification indiquée dans la revendication 1, dans la première étape avec un agent réducteur et dans la deuxième étape, en présence d'un dérivé d'acide carbonique, avec un composé de formule
H₂N-R³ (III),
dans laquelle R³ a la signification indiquée dans la revendication 1, ou
suivant le procédé [B], on fait réagir un composé de formule (II) dans la première étape avec un agent réducteur et dans la deuxième étape avec un composé de formule
QCN-R³ (IV),
dans laquelle R³ a la signification indiquée dans la revendication 1, ou suivant le procédé [C], on fait réagir un composé de formule 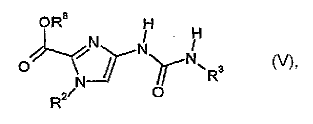 dans laquelle
R² et R³ ont la signification indiquée dans la revendication 1 et
R⁸ représente le méthyle ou l'éthyle,
dans la première étape avec une base et dans la deuxième étape avec un composé de formule
R'-H (VI),
dans laquelle R¹a la signification indiquée dans la revendication 1, en présence de réactifs de déshydratation.

5. Composé selon l'une des revendications 1 à 3, destiné au traitement et/ou à la prévention de maladies.

6. Médicament contenant un composé selon l'une des revendications 1 à 3 en combinaison avec au moins un excipient pharmaceutiquement acceptable non toxique inerte.

7. Utilisation d'un composé selon l'une des revendications 1 à 3 pour fabriquer un médicament destiné au traitement et/ou à la prévention d'infections virales.

8. Utilisation selon la revendication 7, **caractérisée en ce que** l'infection virale est une infection provoquée par le cytomégalovirus humain (CMVH) ou un autre représentant du groupe des virus de l'herpès,

9. Médicament selon la revendication 6, destiné au traitement et/ou à la prévention d'infections virales.

10. Utilisation d'une quantité ayant une efficacité antivirale d'au moins un composé selon l'une des revendications 1 à 3, d'un médicament selon la revendication 6 ou d'un médicament obtenu selon la revendication 7 ou 8 pour fabriquer un médicament destiné à lutter contre les infections virales chez l'homme et chez les animaux.
